(19) Europäisches Patentamt
European Patent Office
Office européen des brevets

(11) EP 3 204 776 B1

(12) EUROPEAN PATENT SPECIFICATION

(45) Date of publication and mention of the grant of the patent:
**04.09.2019 Bulletin 2019/36**

(21) Application number: **15780808.0**

(22) Date of filing: **07.10.2015**

(51) Int Cl.:
***G01N 33/574*** $^{(2006.01)}$ *G01N 33/49* $^{(2006.01)}$

(86) International application number:
**PCT/EP2015/073078**

(87) International publication number:
**WO 2016/055497 (14.04.2016 Gazette 2016/15)**

(54) **METHODS FOR PERSONALIZING PATIENT CANCER THERAPY WITH AN MDM2 ANTAGONIST**

VERFAHREN ZUM PERSONALISIEREN VON PATIENTENKREBSTHERAPIEN MIT EINEM MDM2-ANTAGONIST

PROCÉDÉS POUR PERSONNALISER LA THÉRAPIE ANTICANCÉREUSE D'UN PATIENT PAR UN ANTAGONISTE DE MDM2

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **10.10.2014 US 201462062214 P**
**01.12.2014 US 201462085863 P**

(43) Date of publication of application:
**16.08.2017 Bulletin 2017/33**

(73) Proprietor: **F. Hoffmann-La Roche AG**
**4070 Basel (CH)**

(72) Inventors:
- **BLOTNER, Steven D.**
  **New City, New York 10956 (US)**
- **CHEN, Gong**
  **Rutherford, New Jersey 07070 (US)**
- **JUKOFSKY, Lori**
  **South Plainfield, New Jersey 07080 (US)**
- **NICHOLS, Gwen**
  **New York, New York 10128 (US)**
- **PIERCEALL, William E.**
  **Madison, New Jersey 07940 (US)**
- **REIS, Bernhard**
  **CH-4053 Basel (CH)**
- **RUEGER, Ruediger**
  **82386 Huglfing (DE)**
- **ZHONG, Hua**
  **Short Hills, New Jersey 07078 (US)**

(74) Representative: **Beyermann, Jochen Carl**
**F. Hoffmann-La Roche AG**
**CLP - Patent Department**
**Grenzacherstrasse 124**
**4070 Basel (CH)**

(56) References cited:
**WO-A2-2011/127058** **WO-A2-2014/020502**

- **LONG J ET AL: "Multiple distinct molecular mechanisms influence sensitivity and resistance to MDM2 inhibitors in adult acute myelogenous leukemia", BLOOD, AMERICAN SOCIETY OF HEMATOLOGY, UNITED STATES, vol. 116, no. 1, 8 July 2010 (2010-07-08), pages 71-80, XP002740447, ISSN: 1528-0020, DOI: 10.1182/BLOOD-2010-01-261628 [retrieved on 2010-04-19]**
- **A VAN RHENEN ET AL: "Aberrant marker expression patterns on the CD34+CD38- stem cell compartment in acute myeloid leukemia allows to distinguish the malignant from the normal stem cell compartment both at diagnosis and in remission", LEUKEMIA, vol. 21, no. 8, 24 May 2007 (2007-05-24), pages 1700-1707, XP055172778, ISSN: 0887-6924, DOI: 10.1038/sj.leu.2404754**
- **J J W M JANSSEN ET AL: "Residual normal stem cells can be detected in newly diagnosed chronic myeloid leukemia patients by a new flow cytometric approach and predict for optimal response to imatinib", LEUKEMIA., vol. 26, no. 5, 9 December 2011 (2011-12-09), pages 977-984, XP055237493, US ISSN: 0887-6924, DOI: 10.1038/leu.2011.347**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- F LACOMBE ET AL: "Flow cytometry CD45 gating for immunophenotyping of acute myeloid leukemia", LEUKEMIA, vol. 11, no. 11, 1 January 1997 (1997-01-01), pages 1878-1886, XP055237500, DOI: 10.1038/sj.leu.2400847
- SCHWONZEN ET AL: "Immunophenotyping of surface antigens in acute myeloid leukemia by flow cytometry after red blood cell lysis", LEUKEMIA RESEARCH, NEW YORK,NY, US, vol. 31, no. 1, 11 November 2006 (2006-11-11), pages 113-116, XP005762150, ISSN: 0145-2126, DOI: 10.1016/J.LEUKRES.2006.03.022

**Description**

## BACKGROUND OF THE INVENTION

The TP53 gene encodes a tumor suppressor protein that plays a critical role in the protection during the development of cancers. P53 is crucial in multi-cellular organisms where it regulates cell cycle, and thus acts as a tumor suppressor that is involved in preventing cancer. Further, it is a transcription factor that regulates multiple genes involved in cell cycle control, such as apoptosis, DNA repair and senescence.

Under non-stressed conditions, the level of p53 protein is controlled by MDM2 (Murine Double Minute 2) via a negative feedback loop, wherein MDM2 transcription is driven by p53. MDM2 protein binds to the TP53 protein and blocks its transactivation domain. MDM2 can also function as a p53 ubiquitin ligase, which marks p53 for ubiquitin dependent degradation.

In cells that overexpress MDM2, P53 is inactivated, leading to inefficient growth arrest and apoptosis. Blocking the P53--MDM2 interaction might restore P53 function and could be a novel approach to cancer treatment. Treatment of tumor cells with MDM2 antagonists should enable p53 to mediate its downstream functions, including activation of gene transcription and induction of cell cycle arrest and apoptosis.

TP53 mutations are rare in Acute Myeloid Leukemia (AML) and are generally not considered to be of primary importance in the development of these malignancies. However, MDM2 has been found to be frequently overexpressed in AML, and can enhance the tumorigenic potential and resistance to apoptosis through abrogation of p53 function. It has been found that AML cell lines and 16 primary aAML samples with wild-type p53 responded to MDM2 antagonist (inhibitor) by induction of p53-dependent apoptosis. These findings support the rationale of targeting the p53-MDM2 interaction as a therapeutic strategy for AML.

Based on the proposed mechanism of action of the drug, the presence of functional p53 protein and related pathway effector molecules are required for this class of drugs to be efficacious. Not all patients will have functional p53 proteins and related pathway effector molecules. In order to better determine whether a patient can benefit from therapy, there is a need to discover predictive molecular tests for identifying patients that are most likely to respond to therapy. One approach for assessing potential response to a MDM2 antagonist is to assess whether or not the TP53 gene is mutated. However, this is complicated by the fact that a multitude of mutations can be found in TP53 in cancer. Not all of these mutations will interfere with activity of the p53 protein, further complicating interpretation of TP53 mutational tests. In addition, there is a range of responses to MDM2 antagonists in wild type TP53 cell lines and patients. Also, it has been observed that while many AML patients harboring p53 mutations are non-responsive to MDM2 antagonist therapy, there remain patients with certain p53 mutations who exhibit durable responses to such therapy. Therefore, the ability to predict responsiveness to an MDM2 antagonist from an easily interpretable diagnostic tool is an unmet need in clinical development of MDM2 antagonists.

It has now been found that a biomarker based on the amount of certain MDM2 protein expressing stem cells could provide a means of selecting patients most likely to respond to MDM2 antagonist therapy. This method also has the potential to be combined with other biomarker assessments, including in particular the p53 mutation status, age and the ECOG performance status for algorithms with greater sensitivity and specificity in discriminating clinical endpoints. WO2011/127058 discloses different biomarkers to predict which leukemia patients are likely to benefit from MDM2 inhibitor therapy.

## SUMMARY OF THE INVENTION

In one embodiment, the present invention provides an *in vitro* method for determining a cancer patient's response to treatment with an MDM2 inhibitor, characterized in that the higher the % MDM2 positve cells, detected in a sample obtained from that patient prior to treatment, the higher is the likelihood of said patient to reach a beneficial clinical endpoint, wherein the MDM2 positive (or MDM2 expressing) cells are selected from the group consisting of CD45dim blast cells, CD117+ (c-kit) and CD34+ stem cells, or combinations thereof, and wherein said MDM2 inhibitor is selected from the compounds: 4-{[2R,3S,4R,5S)-4-(4-Cholor-2-fluoro-phenyl)-3-(3-chloro-2-fluoro-phenyl)-4-cyano-5-(2,2-dimethyl-propyl)-pyrrolidine-2-carbonyl]-amino}-3-methoxy-benzoic acid of the formula (B) and 4-{[(2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carbonyl]-amino}-3-methoxy-benzoic acid 1-mPEG-carbonyloxy-ethyl ester (mPEG, average MW, ~2000) of the formula (C) as disclosed herein.

In another aspect the present invention relates to a method for predicting the response of a patient having cancer to therapy, wherein the patients therapy comprises the treatment with a compound which acts as inhibitor of the MDM2-p53 interaction of formula (B) or (C) as disclosed herein, said method comprising the steps of:

a) measuring a level of CD45dim blast cells and/or CD117+ (c-kit) and/or CD34+ stem cells in a sample pre-obtained

from the cancer patient to obtain a value or values representing this level; or

b) measuring the % of MDM2 expressing CD45dim cells and/or CD117+ (c-kit) and/or CD34+ stem cells in a sample pre-obtained from a cancer patient; and

c) comparing the value or values from steps a) and/or b) to a standard value or set of standard values.

Compounds which act as inhibitor of the MDM2-p53 interaction (MDM2-inhibitors, MDM2-antagonists) are known in the art. MDM2-inhibitors and methods for making them are for example disclosed in U.S. Patent 8,354,444 B2, as well as in WO 2007/063013, WO 2010/031713 and WO 2011/098398. Additional MDM2-inhibitors are disclosed in WO 2013/135648.

U.S. Patent 8,354,444 B2, as well as WO 2010/031713, WO 2011/098398 disclose for example compounds of formula I, II and IIa

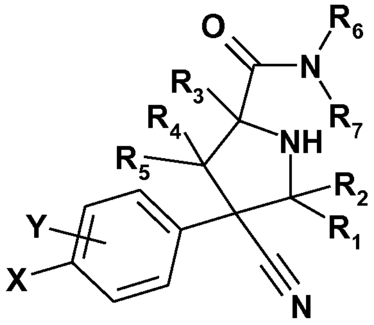

I ,

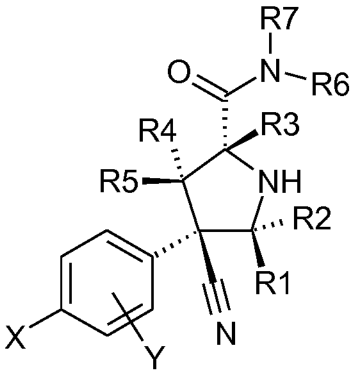

II,

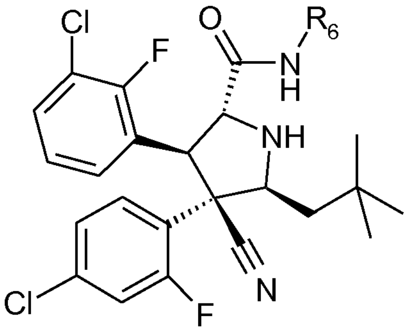

IIa

WO 2013/135648 discloses for example compounds of formula III

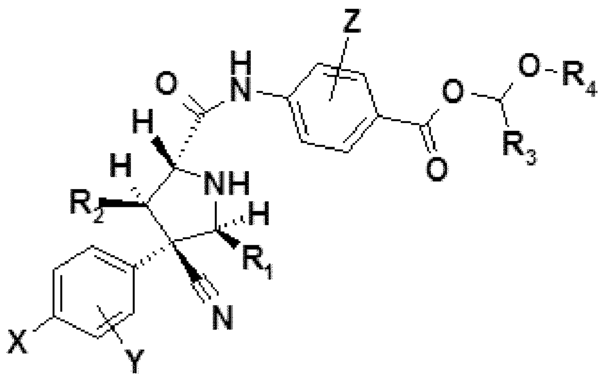

III

In one aspect, the present invention relates to an *in vitro* method for predicting the response of a patient having cancer to therapy, wherein the patients therapy comprises the treatment with a compound which acts as inhibitor of the MDM2-p53 interaction (an "MDM2-inhibitor") as specifically disclosed in the working examples of U.S. Patent 8,354,444 B2, or in WO 2013/135648.

As such, the present invention relates to an *in vitro* method for identifying sensitivity to MDM2 antagonist therapy.

The present invention also relates to the use of CD45dim cells, CD117+ (c-kit) and/or CD34+ stem cells as predictive mechanism for determining the response of a patient suffering from cancer, more particularly acute myeloid leukemia (AML), to treatment with an MDM2-inhibitor as defined herein.

The present invention also relates to any of the above methods for identifying sensitivity to MDM2 antagonist therapy, further comprising detecting a level of at least one gene selected from the group consisting of BAX, RPS27L, EDA2R, XPC, DDB2, FDXR, MDM2, CDKN1A, TRIAP1, BBC3, CCNG1, TNFRSF10B, or CDKN2A and combining that

value with said relative amount of MDM2-protein expressing cells detected according any of the above methods, in order to obtain a combined score "S", and using that combined score "S" as a biomarker for predicting the patient's response to a compound, wherein the compound is an inhibitor of the MDM2-p53 interaction.

## BRIEF DESCRIPTION OF THE FIGURES

**Figure 1:** Identification of living cells, CD45dim/ SSC low blasts and CD34+ blasts by electronic gating.

**Figure 2:** Identification of Mdm2 positive cells in the CD45 dim blast population and in the CD45 dim / CD34 positive blast population. Isotype controls (upper two dot plots) are used to draw the borders for Mdm2 poitivity.

**Figure 3:** MDM2 protein expression in CD45dim (Blast) Cells versus best overall response; **(a)** including mutant p53; and **(b)** without mutant p53.

**Figure 4a**: Correlation of the combined score "S" with response (CR, non-CR) of patients in AML clinical trials (identifier: NP29679, Part 1 and 2), wherein "S" is calculated using the 4-gene signature of Example 2 and the relative amount of MDM2 positive CD45 dim blast cells ($S_{MDM2\text{-}flow}$), including p53 mutant patients:

4a i)

$$S = S_{4\text{-gene-RT-PCR}} \times S_{MDM2\text{-flow}},$$

4a ii)

$$S = S_{4\text{-gene-RT-PCR}} \times \log2(S_{MDM2\text{-flow}}),$$

4a iii)

$$S = S_{4\text{-gene-RT-PCR}} + \log2(S_{MDM2\text{-flow}});$$

and wherein "S" is calculated using the 4-gene signature of Example 2 and the relative amount of MDM2 positive CD45 dim blast cells ($S_{MDM2\text{-}flow}$), including only p53 wild-type (wt) patients:

4a iv)

$$S = S_{4\text{-gene-RT-PCR}} \times S_{MDM2\text{-flow}},$$

4a v)

$$S = S_{4\text{-gene-RT-PCR}} \times \log2(S_{MDM2\text{-flow}}),$$

4a vi)

$$S = S_{4\text{-gene-RT-PCR}} + \log2(S_{MDM2\text{-flow}}).$$

**Figure 4b**: Correlation of combined score "S" with response (CR, non-CR) of patients in AML clinical trials (identifier: NP29679, Part 1 and 2), wherein "S" is calculated using the 3-gene signature of Example 2 and the relative amount of MDM2 positive CD45 dim blast cells ($S_{MDM2\text{-}flow}$), including p53 mutant patients:

4b i)

$$S = S_{3\text{-gene-RT-PCR}} \times S_{MDM2\text{-flow}},$$

4b ii)

$$S = S_{3\text{-gene-RT-PCR}} \times \log2(S_{MDM2\text{-flow}}),$$

4b iii)

$$S = S_{3\text{-gene-RT-PCR}} + \log2(S_{MDM2\text{-flow}});$$

and wherein "S" is calculated using the 3-gene signature of Example 2 and the relative amount of MDM2 positive CD45 dim blast cells ($S_{MDM2\text{-flow}}$), including only p53 wild-type (wt) patients:

4b iv)

$$S = S_{3\text{-gene-RT-PCR}} \times S_{MDM2\text{-flow}},$$

4b v)

$$S = S_{3\text{-gene-RT-PCR}} \times \log2(S_{MDM2\text{-flow}}),$$

4b vi)

$$S = S_{3\text{-gene-RT-PCR}} + \log2(S_{MDM2\text{-flow}}).$$

**Figure 5:** Correlation of a score based on mRNA expression levels for each MDM2 (i), BBC3 (ii), XPC (iii) or CDKN2A (iv) alone with response (CR, non-CR) of patients in AML clinical trials (identifier: NP29679, Part 1 and 2) including p53-wild type (wt) and p53-mutant patients.

## DETAILED DESCRIPTION OF THE INVENTION

In one embodiment, the compound inhibiting the MDM2-p53 interaction (also "MDM2-inhibitor" or "MDM2-antagonist") for use in the method according to present invention is a compound as specifically disclosed in U.S. Patent 8,354,444 B2, or in WO 2013/135648.

Reference compounds as MDM2-inhibitor for use in the method according to present invention is a compound of formula I

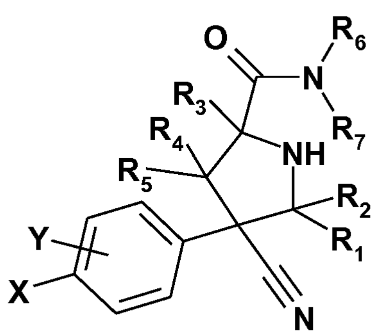

I

wherein

X is selected from the group consisting of H, F, Cl, Br, I, cyano, nitro, ethynyl, cyclopropyl, methyl, ethyl, isopropyl, vinyl and methoxy,

Y is one to four group(s) independently selected from the group consisting of H, F, Cl, Br, I, CN, OH, nitro, lower alkyl, cycloalkyl, lower alkoxy, lower alkenyl, cycloalkenyl, lower alkynyl, aryl, hetereoaryl, hetereocycle, COOR', OCOR', CONR'R", NR'COR", NR"$SO_2$R', $SO_2$NR'R" and NR'R" wherein

R' and R" are independently selected from H, lower alkyl, substituted lower alkyl, lower cycloalkyl, substituted lower cycloalkyl, lower alkenyl, substituted lower alkenyl, lower cycloalkenyl, substituted lower cycloalkenyl, aryl, substituted aryl, hetereoaryl, substituted hetereoaryl, hetereocycle, or substituted hetereocycle.

and in the case of R' and R" may independently link to form a cyclic structure selected from substituted or unsubstituted cycloalkyl, substituted or unsubstituted cycloalkenyl, substituted or unsubstituted heteroaryl or substituted or unsubstituted heterocycle,

one of $R_1$ and $R_2$ is selected from the group consisting of lower alkyl,

substituted lower alkyl, lower alkenyl, substituted lower alkenyl, aryl, substituted aryl, heteroaryl, substituted heteroaryl, heterocycle, substituted heterocycle, cycloalkyl, substituted cycloalkyl, cycloalkenyl, and substituted cycloalkenyl and the other is hydrogen or lower alkyl, $R_3$ is H or lower alkyl,

one of $R_4$ and $R_5$ is selected from the group consisting of lower alkyl, substituted lower alkyl, lower alkenyl, substituted lower alkenyl, aryl, substituted aryl, heteroaryl, substituted heteroaryl, heterocycle, substituted heterocycle, cycloalkyl, substituted cycloalkyl, cycloalkenyl, and substituted cycloalkenyl and the other is hydrogen,

$R_6$ and $R_7$ are selected from the group consisting of $(CH_2)_n$-R', $(CH_2)_n$-NR'R", $(CH_2)_n$-NR'COR", $(CH_2)_n$-NR'$SO_2$R", $(CH_2)_n$-COOH, $(CH_2)_n$-COOR', $(CH_2)_n$-CONR'R", $(CH_2)_n$-OR', $(CH_2)_n$-SR', $(CH_2)_n$-SOR', $(CH_2)_n$-$SO_2$R', $(CH_2)_n$-COR', $(CH_2)_n$-$SO_3$H, $(CH_2)_n$-SONR'R", $(CH_2)_n$-$SO_2$NR'R", $(CH_2CH_2O)_m$-$(CH_2)_n$-R', $(CH_2CH_2O)_m$-$(CH_2)_n$-OH, $(CH_2CH_2O)_m$-$(CH_2)_n$-OR', $(CH_2CH_2O)_m$-$(CH_2)_n$-NR'R", $(CH_2CH_2O)_m$-$(CH_2)_n$-NR'COR", $(CH_2CH_2O)_m$-$(CH_2)_n$-NR'$SO_2$R", $(CH_2CH_2O)_m$-$(CH_2)_n$-COOH, $(CH_2CH_2O)_m$-$(CH_2)_n$-COOR', $(CH_2CH_2O)_m$-$(CH_2)_n$-CONR'R", $(CH_2CH_2O)_m$-$(CH_2)_n$-$SO_2$R', $(CH_2CH_2O)_m$-$(CH_2)_n$-COR', $(CH_2CH_2O)_m$-$(CH_2)_n$-SONR'R", $(CH_2CH_2O)_m$-$(CH_2)_n$-$SO_2$NR'R", $(CH_2)_p$-$(CH_2CH_2O)_m$-$(CH_2)_n$-R', $(CH_2)_p$-$(CH_2CH_2O)_m$-$(CH_2)_n$-OH, $(CH_2)_p$-$(CH_2CH_2O)_m$-$(CH_2)_n$-OR', $(CH_2)_p$-$(CH_2CH_2O)_m$-$(CH_2)_n$-NR'R", $(CH_2)_p$-$(CH_2CH_2O)_m$-$(CH_2)_n$-NR'COR", $(CH_2)_p$-$(CH_2CH_2O)_m$-$(CH_2)_n$-NR'$SO_2$R", $(CH_2)_p$-$(CH_2CH_2O)_m$-$(CH_2)_n$-COOH, $(CH_2)_p$-$(CH_2CH_2O)_m$-$(CH_2)_n$-COOR', $(CH_2)_p$-$(CH_2CH_2O)_m$-$(CH_2)_n$-CONR'R", $(CH_2)_p$-$(CH_2CH_2O)_m$-$(CH_2)_n$-$SO_2$R', $(CH_2)_p$-$(CH_2CH_2O)_m$-$(CH_2)_n$-COR', $(CH_2)_p$-$(CH_2CH_2O)_m$-$(CH_2)_n$-SONR'R", $(CH_2)_p$-$(CH_2CH_2O)_m$-$(CH_2)_n$-$SO_2$NR'R", -COR', -SOR' and $SO_2$R' wherein R' and R" are as above,

m, n and p are independently 0 to 6, and

the pharmaceutically acceptable salts and esters thereof.

Reference compounds of formula I may have a stereochemical structure shown as formula II:

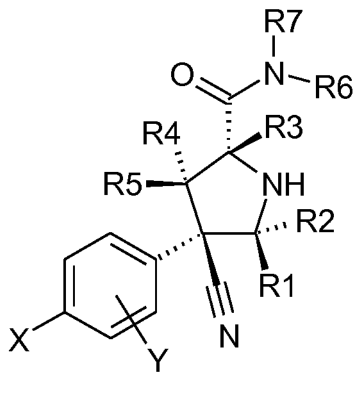

II.

Further reference compound as MDM2-inhibitor for use in the method according to present invention is a compound of formula IIa

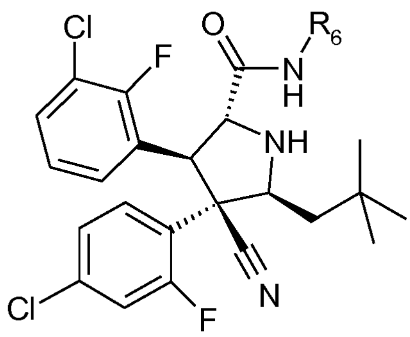

(IIa),

wherein

$R_6$ is $-(CH_2)_n-R'$, and
R' is Cyclohexyl, or
a 5 to 10 membered, mono- or bicyclic aromatic hydrocarbon wherein 1 or 2 carbon atoms may be replaced by N, S or O, and wherein any of the aforementioned cyclohexyl or aromatic hydrocarbon can be substituted once or twice with a group independently selected from lower alkyl, lower-alkenyl, lower-alkynyl, dioxo-lower-alkylene (forming e.g. a benzodioxyl group), halogen, hydroxy, CN, $CF_3$, $NH_2$, N(H, lower-alkyl), N(lower-alkyl)$_2$, aminocarbonyl, carboxy, $NO_2$, lower-alkoxy, thio-lower-alkoxy, lower-alkylsufonyl, aminosulfonyl, lower-alkylcarbonyl, lower-alkylcarbonyloxy, lower-alkoxycarbonyl, lower-alkyl-carbonyl-NH, fluoro-lower-alkyl, fluoro-lower-alkoxy, lower-alkoxycarbonyl-lower-alkoxy, carboxy-lower-alkoxy, carbamoyl-lower-alkoxy, hydroxy-lower-alkoxy, $NH_2$-lower-alkoxy, N(H, lower-alkyl)-lower-alkoxy, N(lower-alkyl)$_2$-lower-alkoxy, lower-alkyl-1-oxiranyl-lower-alkoxy-lower-alkyl, 2-oxo-pyrrolidin-1-yl, (1,1-dioxo)-2-isothiazolidine, 3-lower-alkyl sulfinyl, a substituted or unsubstituted heterocyclic ring, a substituted or unsubstituted aryl ring, a substituted or unsubstituted heteroaryl ring, trifluoro-lower-alkylsulfonylamino-aryl, lower-alkyl sulfonylaminocarbonyl, lower-alkyl sulfonylaminocarbonyl-aryl, hydroxycarbamoyl-phenyl, benzyloxy-lower-alkoxy, mono- or di-lower alkyl substituted amino-sulfonyl and lower-alkyl which can optionally be substituted with halogen, hydroxy, $NH_2$, N(H, lower-alkyl) or N(lower-alkyl)$_2$; and
n is 0 or 1.

Reference compounds of formula IIa are preferred, wherein
$R_6$ is $-(CH_2)_n-R'$, and
R' is phenyl, pyridinyl, pyrazinyl or pyrimidinyl which can be each unsubstituted or once or twice substituted with a substituent independently selected from halogen, C1-6 alkoxy, C1-6 alkyl, hydroxycarbonyl, carboxy, carboxy C1-6 alkoxy, oxo and CN; and
n is 0.

Further reference compounds as MDM2-inhibitor for use in the method according to present invention is a compound of formula III

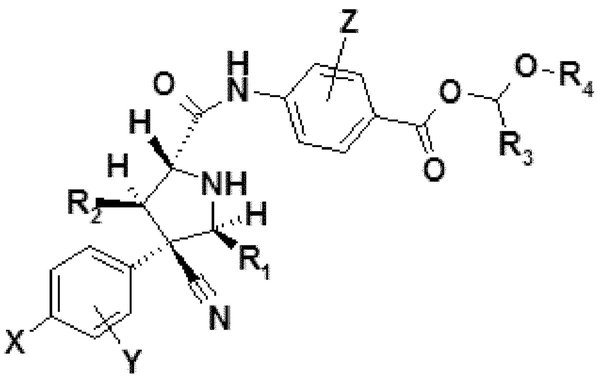

**(III),**

wherein

X is selected from the group consisting of H, F, Cl, Br, I, cyano, nitro, ethynyl, cyclopropyl, methyl, ethyl, isopropyl, vinyl and methoxy,
Y is one to four group(s) independently selected from the group consisting of H, F, Cl, Br, I, CN, OH, nitro, lower alkyl, cycloalkyl, lower alkoxy, lower alkenyl, cycloalkenyl, lower alkynyl,
Z is lower alkoxy,
$R_1$ is selected from the group consisting of lower alkyl, substituted lower alkyl, lower alkenyl, substituted lower alkenyl, aryl, substituted aryl, heteroaryl, substituted heteroaryl, heterocycle, substituted heterocycle, cycloalkyl, substituted cycloalkyl, cycloalkenyl, and substituted cycloalkenyl,
$R_2$ is a substituted phenyl selected from:

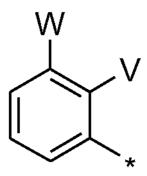 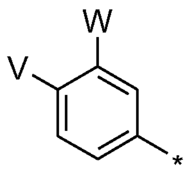 or 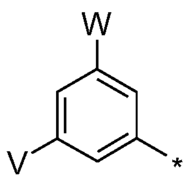 ,

W is F, Cl or Br,
V is H or F,
$R_3$ is selected from the group consisting of hydrogen, lower alkyl or substituted lower alkyl,
$R_4$ is selected from the group consisting of:

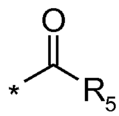 or 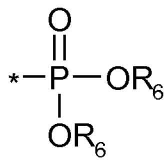

$R_5$ is selected from the group consisting of lower alkyl, substituted lower alkyl, heterocycle, substituted heterocycle, cycloalkyl, substituted cycloalkyl, natural and unnatural amino acids, $-(OCH_2CH_2)_n-OH$, $-(OCH_2CH_2)_n-OCH_3$, $-(NCH_2CH_2)_n-OH$, $-(NCH_2CH_2)_n-OCH_3$ and $-(OCH_2CH_2)_n-OP(O)(OR_6)_2$, wherein
n is from 3 to 60, preferably from 3 to 45,
$R_6$ is hydrogen or benzyl; or
a pharmaceutically acceptable salt or ester thereof.

Reference compounds of formula III are disclosed, wherein
X is selected from H, F or Cl,
Y is selected from H, F or Cl,
$R_1$ is lower alkyl or substituted lower alkyl,
$R_3$ is hydrogen or lower alkyl,
$R_5$ is selected from the group consisting of lower alkyl, substituted lower alkyl, natural and unnatural amino acids, $-(OCH_2CH_2)_n-OH$, $-(OCH_2CH_2)_n-OCH_3$, $-(NCH_2CH_2)_n-OH$,$-(NCH_2CH_2)_n-OCH_3$, $-(OCH_2CH_2)_n-OP(O)(OR_6)_2$, wherein
n is from 3 to 60, preferably from 3 to 45, and
$R_6$ is hydrogen; or
a pharmaceutically acceptable salt thereof.

Furthermore, reference compounds of formula III are disclosed, wherein
X is selected from H, F or Cl;
Y is selected from H, F or Cl;
Z is C1-6 alkoxy;
$R_1$ is C1-6 alkyl;
$R_2$ is

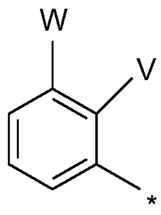

wherein

W is F, Cl or Br;
V is H or F;
$R_3$ is hydrogen or C1-6 alkyl;
$R_4$ is -C(O)-$R_5$; wherein
$R_5$ is selected from the group consisting of -$(OCH_2CH_2)_n$-OH; -$(OCH_2CH_2)_n$-$OCH_3$; and -$(OCH_2CH_2)_n$-OP(O)$(OR_6)_2$, wherein n is from 3 to 60, and $R_6$ is hydrogen; or
a pharmaceutically acceptable salt thereof.
More specifically n is from 3 to 55, more preferably n is from 3 to 45. Reference compounds wherein $R_5$ is -$(OCH_2CH_2)_n$-$OCH_3$ and n is from 40 to 60 are also disclosed.

In the specification where indicated the various groups may be substituted by 1-5 or, preferably, 1-3 substituents independently selected from the group consisting of lower alkyl, lower-alkenyl, lower-alkynyl, dioxo-lower-alkylene (forming e.g. a benzodioxyl group), halogen, hydroxy, CN, $CF_3$, $NH_2$, N(H, lower-alkyl), N(lower-alkyl)$_2$, aminocarbonyl, carboxy, $NO_2$, lower-alkoxy, thio-lower-alkoxy, lower-alkylsufonyl, aminosulfonyl, lower-alkylcarbonyl, lower-alkylcarbonyloxy, lower-alkoxycarbonyl, lower-alkyl-carbonyl-NH, fluoro-lower-alkyl, fluoro-lower-alkoxy, lower-alkoxy-carbonyl-lower-alkoxy, carboxy-lower-alkoxy, carbamoyl-lower-alkoxy, hydroxy-lower-alkoxy, $NH_2$-lower-alkoxy, N(H, lower-alkyl)-lower-alkoxy, N(lower-alkyl)$_2$-lower-alkoxy, lower-alkyl-1-oxiranyl-lower-alkoxy-lower-alkyl, 2-oxo-pyrrolidin-1-yl, (1,1-dioxo)-2-isothiazolidine, 3-lower-alkyl sulfinyl, a substituted or unsubstituted heterocyclic ring, a substituted or unsubstituted aryl ring, a substituted or unsubstituted heteroaryl ring, trifluoro-lower-alkylsulfonylamino-aryl, lower-alkyl sulfonylaminocarbonyl, lower-alkyl sulfonylaminocarbonyl-aryl, hydroxycarbamoyl-phenyl, benzyloxy-lower-alkoxy, mono- or di-lower alkyl substituted amino-sulfonyl and lower-alkyl which can optionally be substituted with halogen, hydroxy, $NH_2$, N(H, lower-alkyl) or N(lower-alkyl)$_2$. Preferred substituents for the cycloalkyl, cycloalkenyl, aryl, heteroaryl and heterocycle rings are halogen, lower alkoxy, lower alkyl, hydroxycarbonyl, carboxy, carboxy lower alkoxy, oxo and CN. Preferred substituents for alkyl are alkoxy and N(lower alkyl)$_2$.

The term "alkyl" refers to straight- or branched-chain saturated hydrocarbon groups having from 1 to about 20 carbon atoms, including groups having from 1 to about 7 carbon atoms. In certain embodiments, alkyl substituents may be lower alkyl substituents. The term "lower alkyl" refers to alkyl groups having from 1 to 6 carbon atoms, and in certain embodiments from 1 to 4 carbon atoms. Examples of alkyl groups include, but are not limited to, methyl, ethyl, n-propyl, i-propyl, n-butyl, s-butyl, t-butyl, n-pentyl, and s-pentyl.

As used herein, "cycloalkyl" is intended to refer to any stable monocyclic or polycyclic system which consists of carbon atoms only, any ring of which being saturated, and the term "cycloalkenyl" is intended to refer to any stable monocyclic or polycyclic system which consists of carbon atoms only, with at least one ring thereof being partially unsaturated. Examples of cycloalkyls include, but are not limited to, cyclopropyl, cyclobutyl, cyclopentyl, cyclohexyl, cycloheptyl, adamantyl, cyclooctyl, bicycloalkyls, including bicyclooctanes such as [2.2.2]bicyclooctane or [3.3.0]bicyclooctane, bicyclononanes such as [4.3.0]bicyclononane, and bicyclodecanes such as [4.4.0]bicyclodecane (decalin), or spiro compounds. Examples of cycloalkenyls include, but are not limited to, cyclopentenyl or cyclohexenyl.

The term "alkenyl" as used herein means an unsaturated straight-chain or branched aliphatic hydrocarbon group containing one double bond and having 2 to 6, preferably 2 to 4 carbon atoms. Examples of such "alkenyl group" are vinyl ethenyl, allyl, isopropenyl, 1-propenyl, 2-methyl-1-propenyl, 1-butenyl, 2-butenyl, 3-butenyl, 2-ethyl-1-butenyl, 3-methyl-2-butenyl, 1-pentenyl, 2-pentenyl, 3-pentenyl, 4-pentenyl, 4-methyl-3-pentenyl, 1-hexenyl, 2-hexenyl, 3-hexenyl, 4-hexenyl and 5-hexenyl.

The term "alkynyl" as used herein means an unsaturated straight-chain or branched aliphatic hydrocarbon group containing one triple bond and having 2 to 6, preferably 2 to 4 carbon atoms. Examples of such "alkynyl group" are ethynyl, 1-propynyl, 2-propynyl, 1-butynyl, 2-butynyl, 3-butynyl, 1-pentynyl, 2-pentynyl, 3-pentynyl, 4-pentynyl, 1-hexynyl, 2-hexynyl, 3-hexynyl, 4-hexynyl and 5-hexynyl.

The term "halogen" as used in the definitions means fluorine, chlorine, bromine, or iodine, preferably fluorine and chlorine.

"Aryl" means a monovalent, monocyclic or bicyclic, aromatic carbocyclic hydrocarbon radical, preferably a 6-10 member aromatic ring system. Preferred aryl groups include, but are not limited to, phenyl, naphthyl, tolyl, and xylyl. Where the aryl group is bicyclic a preferred group is 1,3-dioxo-2,3-dihydro-1H-isoindol-5-yl group.

"Heteroaryl" means an aromatic heterocyclic ring system containing up to two rings. Preferred heteroaryl groups include, but are not limited to, thienyl, furyl, indolyl, pyrrolyl, pyridinyl, pyrazinyl, oxazolyl, thiaxolyl, quinolinyl, pyrimidinyl, imidazole substituted or unsubstituted triazolyl and substituted or unsubstituted tetrazolyl.

In the case of aryl or heteroaryl which are bicyclic it should be understood that one ring may be aryl while the other is heteroaryl and both being substituted or unsubstituted.

"Heterocycle" or "heterocyclic ring"means a substituted or unsubstituted 5 to 8 membered, mono- or bicyclic, non-aromatic hydrocarbon, wherein 1 to 3 carbon atoms are replaced by a hetero atom selected from nitrogen, oxygen or sulfur atom. Examples include pyrrolidin-2-yl; pyrrolidin-3-yl; piperidinyl; morpholin-4-yl and the like which in turn can be substituted. "Hetero atom" means an atom selected from N, O and S.

"Alkoxy, alkoxyl or lower alkoxy" refers to any of the above lower alkyl groups attached to an oxygen atom. Typical lower alkoxy groups include methoxy, ethoxy, isopropoxy or propoxy, butyloxy and the like. Further included within the meaning of alkoxy are multiple alkoxy side chains, *e.g.* ethoxy ethoxy, methoxy ethoxy, methoxy ethoxy ethoxy and the like and substituted alkoxy side chains, e.g., dimethylamino ethoxy, diethylamino ethoxy, dimethoxy-phosphoryl methoxy and the like.

The term "mPEG" as used herein means methoxy polyethylene glycol, which is commercially available (e.g. Sigma-Aldrich or ID Biochem (Korea)). The molecular weight distribution of mPEG may vary according to the manufacturer and/or batch. In one embodiment of the present invention, mPEG has an average molecular weight (MW) of about 1500 Da to about 3000 Da. In another embodiment of the present invention mPEG has an average MW of about 2000 Da and about 2200 Da. Average MW is determined by MALDI-TOF mass spectrometry.

"Pharmaceutically acceptable derivative," such as pharmaceutically acceptable salts & esters, carrier, excipient, means pharmacologically acceptable and substantially non-toxic to the subject to which the particular compound is administered.

"Pharmaceutically acceptable salt" refers to conventional acid-addition salts or base-addition salts that retain the biological effectiveness and properties of the compounds of the present invention and are formed from suitable non-toxic organic or inorganic acids or organic or inorganic bases. Sample acid-addition salts include those derived from inorganic acids such as hydrochloric acid, hydrobromic acid, hydroiodic acid, sulfuric acid, sulfamic acid, phosphoric acid and nitric acid, and those derived from organic acids such as p-toluenesulfonic acid, salicylic acid, methanesulfonic acid, oxalic acid, succinic acid, citric acid, malic acid, lactic acid, fumaric acid, trifluoro acetic acid and the like. Sample base-addition salts include those derived from ammonium, potassium, sodium and, quaternary ammonium hydroxides, such as for example, tetramethylammonium hydroxide. Chemical modification of a pharmaceutical compound (i.e. drug) into a salt is a technique well known to pharmaceutical chemists to obtain improved physical and chemical stability, hygroscopicity, flowability and solubility of compounds. *See, e.g.,* Ansel et al., Pharmaceutical Dosage Forms and Drug Delivery Systems (6th Ed. 1995) at pp. 196 and 1456- 1457.

The term "ECOG scale" or ECOG performance status" means a parameter or criteria developed by scientists and physicians to assess how a patient's disease (cancer) is progressing and affects the daily living abilities of said partient. The scale was developed by the Eastern Cooperative Oncology Group (ECOG) and is well known to a person of skill in the art. For details see e.g. Oken, M.M. et al, Am. J. Clin. Oncol., 1982, 649-655.

A specific reference MDM2-inhibitor in accordance with the present invention is the compound of formula (A), also designated as Compound A (RG7112)

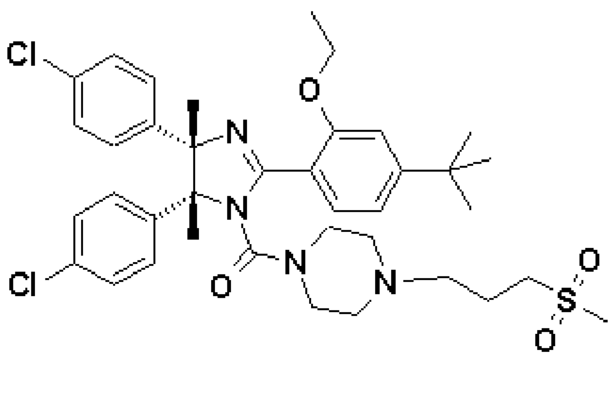

(A).

This compound and methods of making it are for example disclosed in WO 2007/063013.

In one embodiment, an MDM2-inhibitor for use in the method according to present invention is

4-{[(2R,3S,4R,5S)-4-(4-Chloro-2-fluoro-phenyl)-3-(3-chloro-2-fluorophenyl)-4-cyano-5-(2,2-dimethyl-propyl)-pyrrolidine-2-carbonyl]-amino}-3-methoxy-benzoic acid of the formula (B)

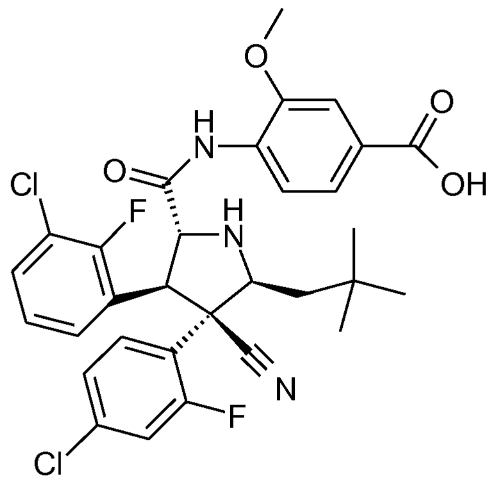

(B).

This compound and methods for making it are for example disclosed in WO 2011/098398.

Another specific reference compound in accordance with the present invention is 2-((1-(4-((2R,3S,4R,5S)-3-(3-Chloro-2-fluorophenyl)-4-(4-chloro-2-fluorophenyl)-4-cyano-5-neopentylpyrrolidine-2-carboxamido)-3-methoxybenzoyloxy)ethoxy)carbonylamino)acetic acid.

Another specific reference compound in accordance with the present invention is 2-(((4-((2R,3S,4R,5S)-3-(3-Chloro-2-fluorophenyl)-4-(4-chloro-2-fluorophenyl)-4-cyano-5-neopentylpyrrolidine-2-carboxamido)-3-methoxybenzoyloxy)methoxy)-carbonylamino)acetic acid.

A further reference compound in accordance with the present invention is 3-Oxo-2,4,7,10,13,16,19-heptaoxaicosyl 4-((2R,3S,4R,5S)-3-(3-chloro-2-fluorophenyl)-4-(4-chloro-2-fluorophenyl)-4-cyano-5-neopentylpyrrolidine-2-carboxamido)-3-methoxybenzoate.

A specific compound in accordance with the present invention is 4-{[(2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carbonyl]-amino}-3-methoxy-benzoic acid 1-mPEG-carbonyloxy-ethyl ester (mPEG, average MW, ~2000).

A specific compound in accordance with the present invention is 4-{[(2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carbonyl]-amino}-3-methoxy-benzoic acid 1-mPEG-carbonyloxy-ethyl ester (mPEG, average MW, ~2200)

A specific reference compound in accordance with the present invention is 4-{[(2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carbonyl]-amino}-3-methoxy-benzoic acid 1-mPEG-carbonyloxy-methyl ester (mPEG, average MW, ~2000).

A specific reference compound in accordance with the present invention is 3-Oxo-2,4,7,10,13-pentaoxatetradecyl 4-((2R,3S,4R,5S)-3-(3-chloro-2-fluorophenyl)-4-(4-chloro-2-fluorophenyl)-4-cyano-5-neopentylpyrrolidine-2-carboxamido)-3-methoxybenzoate;

A specific reference compound in accordance with the present invention is 27-Oxo-2,5,8,11,14,17,20,23,26,28-decaoxatriacontan-29-yl 4-((2R,3S,4R,5S)-3-(3-chloro-2-fluorophenyl)-4-(4-chloro-2-fluorophenyl)-4-cyano-5-neopentylpyrrolidine-2-carboxamido)-3-methoxybenzoate.

A specific reference compound in accordance with the present invention is 4-{[(2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carbonyl]-amino}-3-methoxy-benzoic acid 1-[(2R,3R,4R,5S)-2-((R)-1,2-dihydroxy-ethyl)-4,5-dihydroxy-tetrahydro-furan-3-yloxycarbonyloxy]-ethyl ester.

Another specific reference compound in accordance with the present invention is 4-{[(2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carbonyl]-amino}-3-methoxy-benzoic acid 1-(2-{2-[2-(2-dibenzyloxyphosphoryloxy-ethoxy)-ethoxy] -ethoxy} -ethoxycarbonyloxy)-ethyl ester.

Also, a specific reference compound in accordance with the present invention is 4-{[(2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carbonyl]-amino}-3-methoxy-benzoic acid 1-(2-{2-[2-(2-phosphonooxy-ethoxy)-ethoxy]-ethoxy}-ethoxycarbonyloxy)-ethyl ester.

Also, a specific compound in accordance with the present invention is 4-{[(2R,3S,4R,5S)-3-(3-Chloro-2-fluorophenyl)-4-(4-chloro-2-fluoro-phenyl)-4-cyano-5-(2,2-dimethyl-propyl)-pyrrolidine-2-carbonyl]-amino}-3-methoxy-benzoic acid 1-mPEG-carbonyloxy-ethyl ester (mPEG, average MW, ~2000) of formula (C)

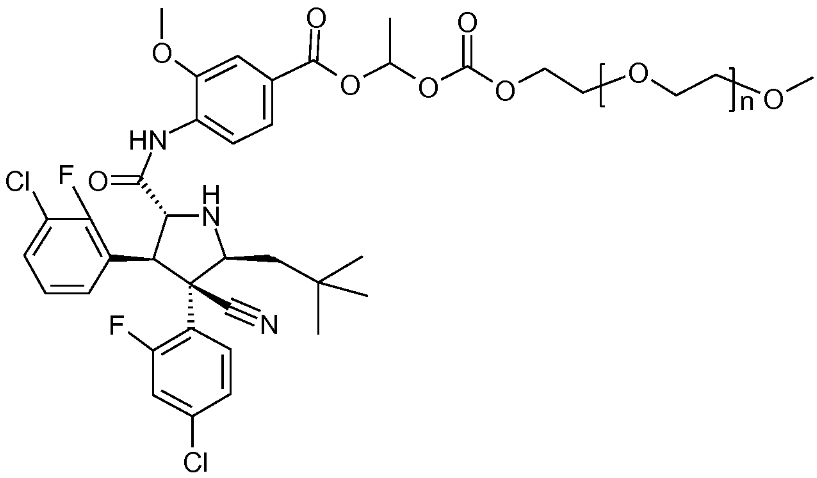

(C).

Average MW: ~2695

This compound and methods for making it are for example disclosed in WO 2013/135648

Also, a specific compound in accordance with the present invention is 4-{[(2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carbonyl]-amino}-3-methoxy-benzoic acid 1-mPEG-carbonyloxy-ethyl ester (mPEG, average MW, ~2200) of formula (D)

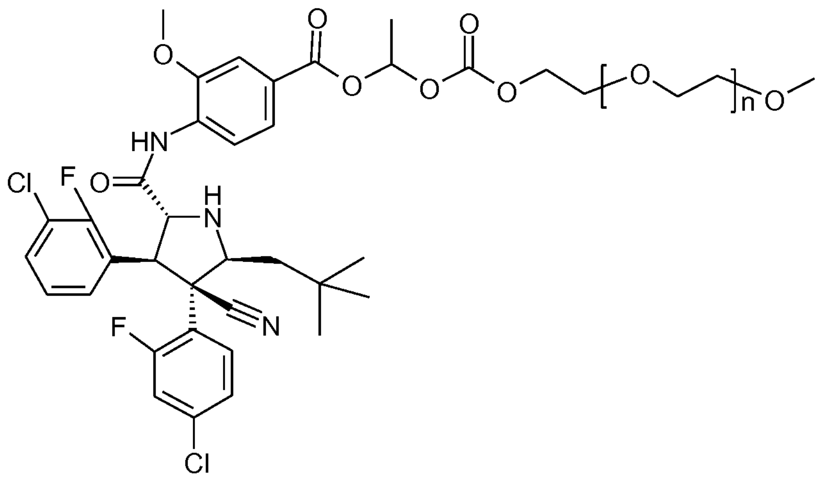

(D)

Average MW: ~2900

This compound and methods for making it are for example disclosed in WO 2013/135648

According to the present invention there is provided a method to predict responsiveness of a patient, with cancer, to a therapy, said method comprising detecting the relative amount of one or several MDM2 protein expressing cell types in a sample obtained from said cancer patient prior to treatment (at baseline), and using said relative amount, or percentage (%) of cells expressing MDM2 protein, as a biomarker for predicting said patient's response to treatment with a medicament, wherein the medicament comprises a compound acting as inhibitor of the MDM2-p53 interaction, as defined herein, and wherein said MDM2 protein expressing cell type, or biomarker, is selected from the group consisting of CD45dim cells, CD117+ (c-kit) and/or CD34+ stem cells, or combinational subsets thereof.

The method in accordance with the present invention is an *in vitro* method.

In another embodiment, the relative amount (%) of each of the MDM2 expressing cell types selected from CD45dim cells, CD117+ (c-kit) and CD34+ stem cells can be used as biomarker for predicting said patient's response to treatment with said MDM2-inhibitor. In another embodiment, combinational subsets of said cell types can also be

used. Within this embodiment, preferred subsets are i) relative amount (%) of MDM2 positive CD117+ (c-kit) and CD45dim cells; or ii) relative amount (%) of MDM2 positive CD34+ and CD45dim cells; or iii) relative amount (%) of MDM2 positive CD34+ and CD117+ and CD45dim cells; or iv) relative amount (%) of MDM2 positive CD45dim cells.

Thus, in another embodiment, the present invention provids a method to predict responsiveness of a patient, with cancer, to a therapy, said method comprising detecting the relative amount of at least CD45dim (blast) cells in a sample obtained from said cancer patient prior to treatment (at baseline), and using said relative amount, or percentage (%) of CD45dim (blast) cells expressing MDM2 protein, as a biomarker for predicting said patient's response to treatment with a medicament, wherein the medicament comprises a compound acting as inhibitor of the MDM2-p53 interaction, as defined herein. Within this and the foregoing embodiments, the "amount" is a relative amount indicated in percent (%) of MDM2 expressing cells of a given type, such as e.g. CD45dim, CD34+ or CD117+, among all cells of that same type detected in the sample obtained from said cancer patient prior to treatment (at baseline). Detection of cells can be carried out by any means known to the skilled artisan, preferably by flow cytometry.

In one embodiment, the present method is a continuous variable measurement. Preferably, the present method is based on a multivariate approach, thus considering one or several sets of variables. In accordance with the present invention, the subsets of MDM2 expressing cells as defined in para [00050] above are preferred variables. In another embodiment, the multivariate approach according to the present invention additionally comprises the patient's age and/or ECOG score as additional variats. The basic principle of multivariate analysis for stratifying patients into groups representing an expected treatment outcome is well known to the person of skill in the art, such as e.g. a clinical oncologist. It is one object of the present invention to define the relative amount of certain MDM2 expressing cells and subsets as defined herein (see e.g. para [00050]) alone or together with the patient's age and/or ECOG score as variants in said analysis.

In another embodiment, the present invention identifies a threshold (or cut-off) value or set of values, such as for example a range, characterized by the amount of MDM2 protein expressing CD45dim (Blast) cells based on clinical data (Study No. NP28679, Part 1 and 2) that identify patients most likely to respond to MDM2 antagonist therapy. The amount is a relative amount indicated in percent (%) of MDM2 expressing cells among all CD45 dim blast cells. The amount is preferably detected by flow cytometry, and is characterized by at least 45%, preferably at least 50% MDM2 protein expressing CD45dim (Blast) cells. Patients with MDM2 protein expressing CD45dim (Blast) cells above those levels are most likely to respond to treatment with an MDM2 inhibitor (MDM2 antagonist), i.e. to reach a beneficial clinical endpoint. The term "response" is further defined herein. The relative amount of said MDM2 expressing CD45dim blast cells is measured in a sample obtained from a cancer patient prior to treatment, and therefore has the potential to serve as a prognostic and/or predictive biomarker for the treatment of said cancer patient with an MDM2 inhibitor as defined herein. In another embodiment, the threshold value for the specific subsets of cells as defined under i) to iv) in para [00050] above is 10%; or 15% or 20%. Patients with a relative amount of MDM2 protein expressing cells above those levels are most likely to respond to treatment with an MDM2 inhibitor (MDM2 antagonist), i.e. to reach a beneficial clinical endpoint.

In one embodiment, the cancer (or "neoplastic disease") is selected from the group consisting of breast cancer, prostate cancer, cervical cancer, ovarian cancer, gastric cancer, colorectal cancer (i.e. including colon cancer and rectal cancer), pancreatic cancer, liver cancer, brain cancer, neuroendocrine cancer, lung cancer, kidney cancer, hematological malignancies, melanoma and sarcomas. In another embodiment, the cancer is selected from the group consisting of hematological malignancies, including leukemias, lymphomas, and multiple myeloma. In yet another embodiment, the cancer is acute myeloid leukemia (AML), or Myelodysplatic Syndrome (MDS), or Myeloproliferative Neoplams (MPN).

The term "response" as used herein means meeting any clinical endpoint. Preferably the clinical endpoint is beneficial for the patient, when for example compared to placebo or standard of care. In one embodiment clinical endpoint, or response, as used herein means Overall Survival (OS), Progression Free Survival (PFS) or Event Free Survival (EFS). In another embodiment the term response means Complete Response (CR), partial response (PR), Complete Response with incomplete platelet recovery (CRi), Hematologic Improvement (HI), or Morphologic Leukemia-Free State (MLFS).

The term "MDM2 expressing cells" or "MDM2 positive cells" or "MDM2 protein expressing cells" means a cell type selected from the group consisting of MDM2 protein expressing CD45dim (blast) cells, CD34+ and CD117+ (c-kit) stem cells.

A "relative amount" of MDM2 protein expressing cells means the percent (%) of MDM2 protein expressing cells among all cells of the same type of cells as defined above. In one embodiment, the term "relative amount" means from about 30% to about 100%, or from about 45% to about100% MDM2 positive cells among all cells of the same type detected in the patient's sample. In another embodiment, the term "relative amount" means from about 45% to about 90%, or from about 55% to about 85%, or from about 65% to about 80%, or from about 70% to about 75% MDM2 protein expressing cells. In another embodiment the term "relative amount" means at least about 45%, or at least about 55% or at least about 65%, or at least about 75% MDM2 protein expressing cells. In another embodiments, the relative amounts indicated above refer to the amounts of MDM2 positive CD45dim (blast) cells, and the amount of MDM2 positive

CD45dim (Blast) cells is relative to all CD45 blast cells in the sample. If several types of cells (subsets) are used for analysis, the term "relative amount" can also mean the percentage of MDM2 expressing cells based on the total number of cells within said subset.

The relative amounts of said MDM2 protein expressing cells can be measured in any suitable sample obtained from said cancer patient prior to treatment (at baseline). In one embodiment, said sample is a blood sample. In another embodiment, said sample is a bone marrow sample. Any technique know to the person of skill in the art is applicable to detect said relative amount of said MDM2 protein expressing cells. In one embodiment the relative amount of said MDM2 protein expressing cells, and in particular of CD45dim (Blast) cells, is detected by flow cytometry.

In one embodiment, the patient is a person suffering from cancer as defined herein. In another embodiment, the patient suffers from a hematological disaorder, such as for example AML.

In another embodiment, the present invention provides a method for determining a cancer patient's response to treatment with an MDM2 inhibitor, comprising the following steps:

a) measuring the relative amount of MDM2 protein expressing cells, in a sample obtained from the patient;
b) comparing said relative amount from the patient to standard values, for example values obtained from a patient with the same cancer.

Within this embodiment, the sample in a) is obtained prior to start of treatment if the present method is applied as a predictive method for a patient's response to treatment; and the MDM2 protein expressing cells in b) are selected from one or several of the group consisting of CD45dim (blast) cells, CD117+ (c-kit) and CD34+.

In another embodiment, the present invention provides an *in vitro* method of identifying a patient suffering from cancer as likely to respond to a therapy comprising an MDM2 inhibitor, the method comprising,

a) measuring the relative amount of MDM2 protein expressing cells in a sample obtained from that patient prior to treatment;
b) comparing said relative amount to a reference level; and
d) identifying said patient as more likely to respond to the therapy comprising said MDM2 inhibitor when the relative amount of MDM2 protein expressing cells in the sample is above said reference level, and wherein
the MDM2 protein expressing cells in a) are selected from one or several of the group consisting of CD45dim (blast) cells, CD117+ (c-kit) and CD34+.

Within these embodiments, a relative amount obtained in a) above the reference level indicates a patient's high likelihood to respond to treatment with an MDM2 inhibitor, whereas a relative amount below said level indicates that said patient is less likely to respond to that treatment.

The performance of the MDM2 antagonist predictive CD45 dim (Blast) cells signature is tested in the clinical setting with specimens from the NP28679 leukemia trial (Figure 3). 30 AML patients treated as part of a dose-escalation study provided whole blood specimens that were analyzable by flow cytometry. The clinical response endpoint was stratified into 5 categories: Complete Response (CR), Complete Response with incomplete platelet recovery (CRi), Morphologic Leukemia-Free State (MLFS), Partial Response (PR), Hematologic Improvement (HI), and Progressive Disease (PD). Blood leukemia samples were collected at baseline screening. Samples were analyzed by flow cytometry and characterized for CD45dim blast cells and then within the subset of these cells further characterized for % of CD45 dim cells scoring as positive for MDM2 protein expression. Baseline % cell positivity for MDM2 was then tested for significant association between responders (CR, CRi, MLFS) and non-responders (PR, HI, PD).

Thus, in one embodiment, the present invention provides a method for determining a cancer patient's response to treatment with an MDM2 inhibitor, characterized in that the higher the % MDM2 positve CD45 dim blast cells, detected in a sample obtained from that patient prior to treatment, the higher is the likelihood of said patient's response to said treatment as for example indicated by ROC (AUC) values and analyses, well known to the person of skill in the art of clinical pharmacology.

In another embodiment, the present invention provides a method for determining a cancer patient's response to treatment with an MDM2 inhibitor, comprising the following steps:

a) measuring the relative amount of MDM2 protein expressing CD45dim (Blast) cells, in a sample obtained from the patient;
b) comparing said relative amount from the patient to standard values, for example values obtained from a patient with the same cancer.

The sample in a) is obtained prior to start of treatment if the present method is applied as a predictive method for a patient's response to treatment.

In another embodiment, the present invention provides an *in vitro* method of identifying a patient suffering from cancer as likely to respond to a therapy comprising an MDM2 inhibitor, the method comprising,

a) measuring the relative amount of MDM2 protein expressing CD45dim (Blast) cells in a sample obtained from that patient prior to treatment;
b) comparing said relative amount to a reference level; and
d) identifying said patient as more likely to respond to the therapy comprising said MDM2 inhibitor when the relative amount of MDM2 protein expressing CD45dim (Blast) cells in the sample is above said reference level.

In one embodiment, the relative amount obtained in a) above the reference level indicates a patient's high likelihood to respond to treatment with an MDM2 inhibitor, whereas a relative amount below said level indicates that said patient is less likely to respond to that treatment.
In one embodiment, the sample obtained in a) is blood leukemia sample, or a bone marrow biopsy sample.
In one embodiment, the MDM2-inhibitor is a compound as specifically disclosed in U.S. Patent 8,354,444 B2, or in WO 2013/135648; or a compound according to formulae (B), or (C).
In another embodiment, the MDM2 inhibitor is the compound 4-{[(2R,3S,4R,5S)-4-(4-Chloro-2-fluoro-phenyl)-3-(3-chloro-2-fluoro-phenyl)-4-cyano-5-(2,2-dimethyl-propyl)-pyrrolidine-2-carbonyl]-amino}-3-methoxy-benzoic acid, of the formula (B)

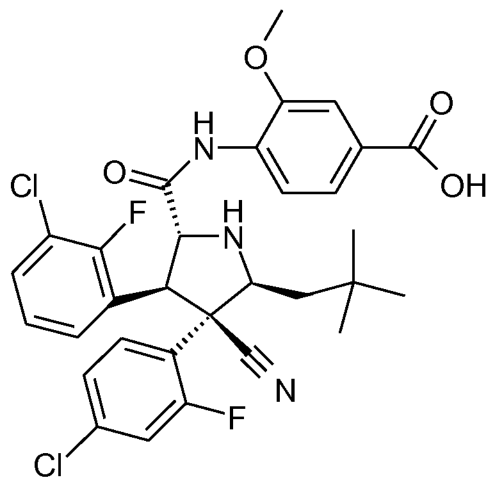

(B).

In yet another embodiment, the MDM2 inhibitor is the compound 4-{[(2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carbonyl]-amino}-3-methoxy-benzoic acid 1-mPEG-carbonyloxy-ethyl ester (mPEG, average MW, ~2000), of the formula (C)

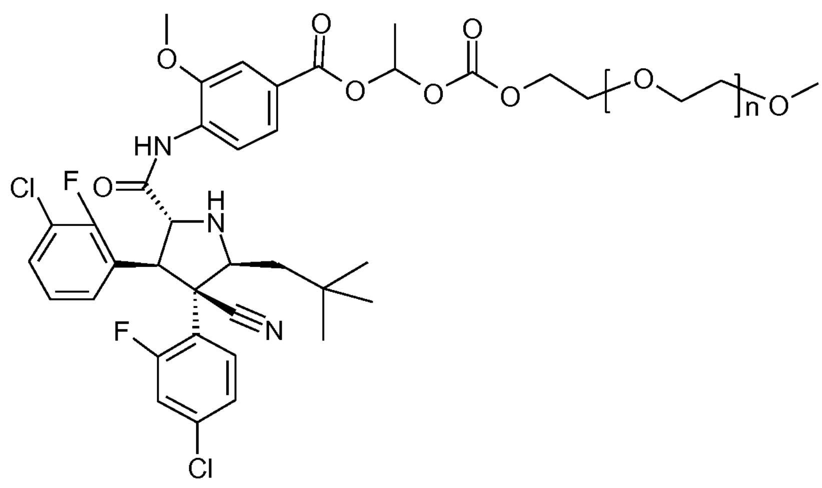

(C),

(Average MW: ~2695).

In yet another embodiment, the MDM2 inhibitor is the compound 4-{[(2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carbonyl]-amino}-3-methoxy-benzoic acid 1-mPEG-carbonyloxy-ethyl ester (mPEG, average MW, ~2200) of the formula (D)

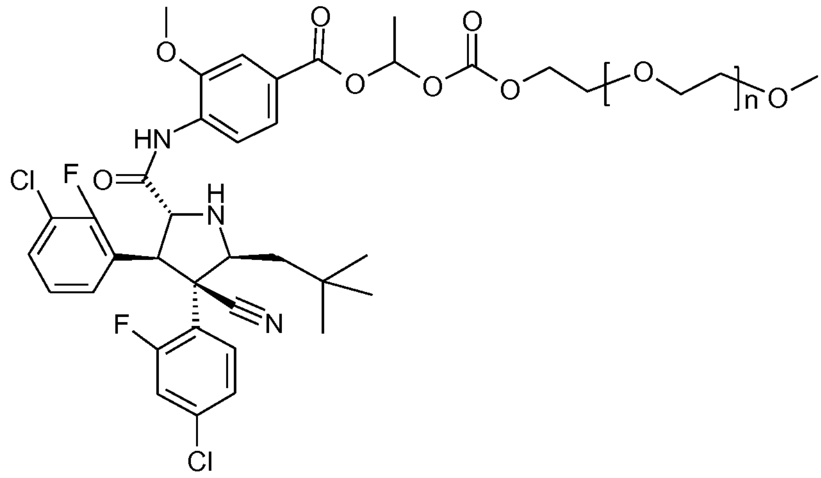

(D),

(Average MW: ~2900).

The MDM2-inhibitors for use in accordance with the present invention are known to the person of skill in the art. The compound of formula (B), methods for making it as well as pharmaceutical preparations containing it are for example disclosed in US Patent No. 8,354,444. The compounds of formula (C), or (D) and their preparation are for example disclosed in WO2013/135648. For the purpose of administering said MDM2-inhibitors to patients, these compounds can be processed as pharmaceutical preparations according to techniques well known to a person of skill in drug delivery.

According to the present disclosure compound (B) is administered as an oral dosage form wherein it is molecularly dispersed in its amorpheous form. In another case, compound (B) is administered in a unit dose solid formulation, characterized in that a solid dispersion obtained by spray drying of the compound (B) together with Copovidone (PVP VA 64) representing about 80% wt/wt of the kernel weight is further blended with a filler (6.8% up to 10.8% of kernel weight), preferably selected from mannitol, microcrystalline cellulose, lactose monohydrate or silicon dioxide; one or two disintegrants (4% wt/wt of kernel weight) selected from croscarmellose sodium or crospovidone; a glidant (1% wt/wt of kernel weight) preferably colloidal silicon dioxide; and a lubricant (0.2% wt/wt of kernel weight) magnesium stearate, using a tumble mixer. The above dosage form, as well as other applicable dosage forms for compound (B) are for example disclosed in WO2014/114575.

Pharmaceutical preparations for compounds of formuala (C), or (D) include those suitable for oral, nasal and/or parenteral or intravenous administration. The formulations may conveniently be presented in unit dosage form and may be prepared by any methods well known in the art of pharmacy. In one embodiment, the compound of formula (C) or (D) is provided in a stable lyophilized formulation for intravenous administration comprising from about 0.1 mg to about 100 mg of compound (I), from about 10mM to about 100mM of a buffering agent, from about 25 mg to about 125 mg of a lyophilization bulking agent and an isotonicity builder. The resultant formulation should have a pH of about 5-7 via adjustment with HCl or NaOH. In another embodiment, the compound of formula (C) or (D) is dissolved in 0.9% sodium chloride in sterile water by vortexing, then filtered through a filter into a septum sealed vial for intravenous administration. are preferably administered as lyophilized formulation for intravenous administration. These preparations, as well as other applicable preparations for compounds of formula (C), or (D) are for example disclosed in WO 2014/206866.

Dosages and dosage schedules for administration of the present MDM2-inhibitors to patients are likewise known to the person of skill in the art and are for example disclosed in WO2013/139687 or US Patent Application No. 14/217929 (published as US 20140200255 A1). In accordance with the present invention, compounds of formula III, and in particular of formula (C) and (D) are prodrugs of the compound of formula (B). The compounds of formula III, in particular of formula (C) and (D) will thus be administered in a way to achieve an effective dose of the compound of formula (B) in the patients blood plasma. Thus, in one embodiment, the medicaments according to the present invention comprise compounds of formula (C) or (D) characterized in that they are dosed in such way as to deliver the compound of formula (B) in an amount of from about 50 to about 3000 mg/day, or from about 80 to about 2500 mg/day, or from about 80 to about 1600 mg/day, or from about 200 to about 1600 mg/day, or from about 400 to about 1600 mg/day, or from about 400 to about 1200 mg/day, or from about 400 to about 1000 mg/day, or from about 400 to about 800 mg/day,

or from about 400 to about 600 mg/day for an administration period of up to about 7 days, preferably up to about 5 days, on days 1-7, or preferably days 1-5, of a 28 day treatment cycle, followed by a rest period of from about 21 to about 23 days, preferably up to about 23 days. The daily dosage, i.e. the amount of compound (B) expressed in mg/day, can be administered as a single dose (qd) or in two doses (BID). When two doses are given, they are preferably administered in equal amounts, once in the morning and once in the afternoon. In another embodiment, the present medicaments comprise compounds of formula (C) or (D), characterized in that they are dosed in such way as to deliver the compound of formula (B) in an amount of from about 200 to about 1200 mg/day, or from about 400 to about 1200 mg/day, for an administration period of up to 5 days, on days 1-5, of a 28 day treatment cycle, followed by a rest period of 23 days.

The compound of formula (B) can also be directly administered, i.e. not via the prodrugs of formula III, or (C), or (D), and using the oral dosage forms as described above. Thus in another embodiment of the present invention, the compound of formula (B) is administerd using a solid, oral dosage form, for example a film coated tablet, providing compound of formula (B) in its amorpheous form and in an amount of from about 200 to about 1200 mg/day, or from about 400 to about 1200 mg/day, for an administration period of up to 5 days, on days 1-5, of a 28 day treatment cycle, followed by a rest period of 23 days.

The medicament comprising an MDM2 inhibitor as defined herein may be administered in combination with another therapy known for a given type of cancer. For example, in case of treatment of AML said MDM2-inhibitor is preferably combined with cytarabine or with cytarabine and anthracycline. A therapeutically effective amount (or "effective amount") of cytarabine in accordance with this invention means an amount effective to achieve the synergistic, i.e. more than additive effect. Since cytarabine is used as the backbone therapy for AML for many years, a lot of information is available to the person of skill in the art, for example a clinical physician, about effective and tolerated doses in humans. It has for example been found that cytarabine can be dosed as single agent in the treatment of AML (induction regimen) in high amounts, such as amounts up to 3 g/m2 (intravenous) over 2 hours every 12 hours days 1 to 6. A review about the use of cytarabine in the treatment of leukemias is for example provided in "Nicholas D. Reese, Gary J. Schiller; Curr Hematol Malig Rep, 2013, 8:141-148." In certain combination therapies (e.g. induction therapy of acute non-lymphocytic leukemia), the usual cytarabine dose in combination with other anti-cancer drugs is 100 mg/m2/day by continuous iv infusion (Days 1-7) or 100 mg/m2 iv every 12 hours (Days 1-7). (see for example www.hospira.com).

Likewise, the skilled person, such as a clinical oncologist, is well aware of the availability and use of anthracycline based cancer therapies. An extensive review of such treatment option is for example provided in: G. Minotti et al, Anthracyclines: Molecular Advances and Pharmacologic Developments in Antitumor Activity and Cardiotoxicity, Pharmacological Reviews, 2004, Vol. 56 No. 2, 185-229.

According to another disclosure, the MDM2 inhibitor according to the present invention, preferably the compound of formulae formula III, or (B), or (C), or (D) is administered in combination with the compound cobimetinib for the treatment of cancer as defined herein. Dosage forms, dosages and dosage schemes for cobimetinib are for example available from the published coBRIM Phase III trial (ClinicalTrials.gov identifier NCT01689519).

The information obtained on MDM2 positive cells as defined herein, in particular MDM2 positive CD45dim (blast) cells, can be used in the context of p53 gene mutation status to more accurately discriminate patient responses/outcomes to treatment. The patient's p53 gene mutation status may serve as an additional marker to detect said patients response to treatment with an MDM2 inhibitor according to the present invention with improved sensitivity and/or specificity. Therefore, in yet another embodiment, the present invention provides any of the methods for measuring the % of MDM2 expressing cells as defined herein, in particular CD45dim (blast) cells, in a samle obtained from a cancer patient, as disclosed herein before, further comprising the step of detecting p53 gene mutation status in said patient's sample. Within this embodiment, the p53 gene mutation status is preferably wild-type. However, detection of p53 gene mutations should not in general be understood as an exclusion criterion for that patient's likelihood to respond to - or benefit from treatment with an MDM2-inhibitor as disclosed herein. Thus, in yet another embodiment the present invention provides a combined, multivariate analysis of % MDM2 expressing cells as defined herein, in particular CD45dim (blast) cells, and p53 mutation status in order to provide a more refined prediction of said patient's likelihood to meet a beneficial clinical endpoint, i.e. to respond to treatment.

## Method of Treatment

The present disclosure also involves a method of treatment, wherein the relative amount of MDM2 protein expressing cells as defined herein, in particular CD45dim (blast) cells, is first detected from a sample, preferably a blood- or bone marrow sample, obtained from a patient suffering from cancer for sensitivity relative to a standard level or set of standard levels or pre-treatment initiation levels and then an MDM2-inhibitor is administered. Preferably said MDM2-inhibitor is a compound of general formula I, II, III, or a pharmaceutically acceptable derivative thereof as defined above, more preferably a compound of formula IIa or III or a pharmaceutically acceptable derivative thereof. Said compounds may be administered in a pharmaceutical composition, as is well known to a person skilled in the art. Compositions for administration, such as nasal, buccal, rectal or, especially, oral administration, and for parenteral administration, such

as intravenous, intramuscular or subcutaneous administration, to warm-blooded animals, especially humans, are especially preferred. More particularly, compositions for intravenous administration are preferred.

Within this disclosure, a compound of general formula I, II or III, preferably IIa or III, or a pharmaceutically acceptable derivative thereof can be administered alone or in combination with one or more other therapeutic agents. Possible combination therapy may take the form of fixed combinations, or the administration of a compound of the invention and one or more other therapeutic agents which are staggered or given independently of one another, or the combined administration of fixed combinations and one or more other therapeutic agents.

Also within this disclosure, a compound of general formal I, II or III, preferably IIa or III, or a pharmaceutically acceptable derivative thereof can, besides or in addition, be administered especially for tumour therapy in combination with chemotherapy (cytotoxic therapy), targeted therapy, endocrine therapy, radiotherapy, immunotherapy, surgical intervention, or a combination of these. Long-term therapy is equally possible as is adjuvant therapy in the context of other treatment strategies, as described above. Other possible treatments are therapy to maintain the patient's status after tumour regression, or even chemo-preventive therapy for example in patients at risk.

## Kit and Device

In one aspect the present disclosure relates to a kit and in another aspect to a device for predicting the response, preferably of a cancer in a subject (or patient), to a compound of general formula I, II, III; or IIa or III; or (A), (B), (C) or (D) or a pharmaceutically acceptable derivative thereof as defined herein, said kit comprising means, tools or devices for sample collection, especially for collection of blood- or bone marrow samples and instructions how to detect relative amounts of MDM2 protein expressing cells as defined herein, in particular CD45dim (blast) cells, in said samples.

The kit and device may also preferably comprise a comparator module which comprises a standard value or set of standard values to which the level of said MDM2 protein expressing cells as defined herein, in particular CD45dim (blast) cells, in the sample is compared.

In another disclosure, there is provided a kit for predicting the response to treatment with a medicament comprising a compound which acts as inhibitor of the MDM2-p53 interaction, comprising;

a) means for obtaining a sample, preferably a blood- or bone marrow sample from a patient suffering from cancer, in particular a hematological disorder such as leukemia, and in particular AML;
b) instructions how to detect the relative amount of one or several of MDM2 protein expressing cell types as defined herein, in particular CD45dim (blast) cells, from that sample, preferably by using flow cytometry, and optionally also detecting additional variats selected from the p53 gene mutation status, age and/or ECOG score of said patient;
c) a comparator module, comprising standard values and instructions how to use them; and
d) a medicament comprising a compound which acts as inhibitor of the MDM2-p53 interaction.

In yet another disclosure there is provided a kit as described above, wherein the compound acting as inhibitor of the MDM2-p53 interaction is a compound formula I, II, III; or IIa or III; or (A), (B), (C) or (D) or a pharmaceutically acceptable derivative thereof as defined herein. The standard value or set of standard values may be determined as described above.

In still another embodiment, there is provided the use of MDM2 protein expressing cells as defined herein, in particular CD45dim (blast) cells, as a biomarker to predict a patients response to treatment with an MDM2 inhibitor (antagonist).

## Combination with predictive gene signatures

In another embodiment the present method, i.e. the detection of the relative amount (%) of MDM2 protein expressing cells according to any embodiment as defined herein before, can be combined with the assessment of a gene signature detected from samples of the same patient at baseline as another co-variate. Suitable gene signatures and methods for obtaining them are for example disclosed in WO 2015/000945. Such combination is done by applying specific algorythms (multivariate analysis) which may provide more accurate prediction of a ptients clinical benefit from treatment with an MDM2- inhibitor as defined herein. Improved accuracy can for example be demonstrated by increase of AUC (i.e. from 0.93 to 0.95) and an increase in p-values from $10^{-3}$ to $10^{-5}$ for the multivariate analysis, compared to any of the already strong biomarkers (MDM2 expressing cells; or gene signature) alone.

Therefore, in one embodiment, the present invention provides a method to predict responsiveness of a patient with cancer to a therapy, said method comprising a) taking one or several samples from said patient; b) measuring the relative amount (%) of at least one MDM2-protein expressing cell type in said sample obtained in step a); c) detecting the expression level of at least one gene according to Table 1 in a sample obtained in step a); d) applying a multivariate analysis using the information of a) and b) in order to calculate a value (combined score "S"); e) comparing said value

for "S" to a standard value (or reference value); and f) identifying said patient as more likely to respond to the therapy when the value of "S" is above said standard value; wherein said therapy comprises administering to said patient a compound acting as inhibitor of the MDM2-p53 interaction (MDM2-inhibitor) as defined herein before.

**TABLE 1:** Significant gene expression predictors for treatment with MDM2-inhibitor

| Ensg.id | Gene | Cor | Pvalue | Mean (Sen) | SD (Sen) | Mean (Res) | SD (Res) | FC | Annotation |
|---|---|---|---|---|---|---|---|---|---|
| ENSG00000087088 | BAX | -0.47 | 9.56E-23 | 5.12 | 0.63 | 4.48 | 0.59 | 1.56 | apoptosis |
| ENSG00000185088 | RPS27L | -0.45 | 2.83E-23 | 4.23 | 0.80 | 3.01 | 0.72 | 2.32 | apoptosis |
| ENSG00000131080 | EDA2R | -0.43 | 1.36E-24 | 0.28 | 3.59 | -4.98 | 2.36 | 38.23 | P53-related |
| ENSG00000154767 | XPC | -0.42 | 5.33E-24 | 3.56 | 0.44 | 2.72 | 0.62 | 1.79 | DNA repair |
| ENSG00000134574 | DDB2 | -0.41 | 4.72E-24 | 4.65 | 0.78 | 3.78 | 0.82 | 1.83 | DNA repair |
| ENSG00000161513 | FDXR | -0.41 | 3.59E-24 | 4.13 | 0.95 | 3.00 | 1.15 | 2.20 | P53-related |
| ENSG00000135679 | MDM2 | -0.39 | 1.12E-24 | 4.63 | 0.87 | 3.60 | 0.64 | 2.04 | |
| ENSG00000124762 | CDKN1A | -0.39 | 1.04E-24 | 5.51 | 1.59 | 3.54 | 2.01 | 3.92 | cell cycle arrest |
| ENSG00000170855 | TRIAP1 | -0.38 | 8.10E-25 | 5.53 | 0.49 | 5.04 | 0.49 | 1.40 | apoptosis |
| ENSG00000105327 | BBC3 | -0.34 | 9.26E-26 | 2.05 | 1.18 | 0.65 | 1.41 | 2.64 | apoptosis |
| ENSG00000113328 | CCNG1 | -0.31 | 1.47E-26 | 5.65 | 0.69 | 4.79 | 0.80 | 1.81 | cell cycle arrest |
| ENSG00000120889 | TNFRSF10B | -0.31 | 1.37E-26 | 4.69 | 1.02 | 3.50 | 1.41 | 2.28 | apoptosis |
| ENSG00000147889 | CDKN2A | 0.28 | 2.38E-47 | -1.71 | 3.79 | 1.35 | 4.26 | 0.12 | MDM2-related |

Within this embodiment, the method is an *in vitro* method. The patient is a cancer patient, and the term "cancer" is defined as herein before. The sample for measurements in steps b) and c) can be the same or different, and is as defined herein before. Preferably said same or different sample is a blood - and/or bone marrow sample, taken from said patient prior to start of treatment (at baseline). The terms "relative amount" and "MDM2 expressing cell types" in step b) are as defined herein before. Preferably, MDM2 protein expressing cell types are selected from CD45dim cells, CD117+ (c-kit) and CD34+ stem cells. The relative amount of one single cell type or combinational subsets of said cell types can be used, as also defined herein before. Measurement of said cell type(s) is carried out as defined above, preferably by flow cytometry. The detection of "at least one gene" in method step c) comprises detection of one single or combined levels comprising 2, or 3, or 4, or 5, or 6, or 7, or 8, or 9, or 10, or 11, or 12, or detection of all 13 genes according to Table 1. Detection of genes preferably means detection of mRNA expression levels for example by RT-PCR (real time polymerase chain reaction) or array technology as disclosed in WO 2015/000945, and as also known to the skilled person. When mRNA expression levels at baseline are measured by RT-PCR, the patient's signature score is calculated from the sum of relative expression levels ($2^{delta\text{-}CP}$) measured at baseline (i.e. prior to treatment), multiplied by the observed direction in-vitro. The combined score "S" obtained in step d) is construed based on multiplication or addition of the value obtained from MDM2 flow measurements obtained in b) with the gene signature scores obtained in c). Preferably said combined score "S" is calculated based on multiplication or addition of MDM2 flow measurements obtained in b) with a 3-gene or 4-gene signature scores according to the algorythms listed in Table 2.

**TABLE 2:** Algorithms for calculating the combined score "S"

| No. | Name | Description |
|---|---|---|
| **1** | 3gscore.times.MDM2.flowc | 3-gene-signature-score * MDM2.flowc |
| **2** | 3gscore.times.log2.MDM2.flowc | 3-gene-signature-score * log2(MDM2.flowc) |
| **3** | 3gscore.plus.log2.MDM2.flowc | 3-gene-signature-score + log2(MDM2.flowc) |
| **4** | 4gscore.times.MDM2.flowc | 4-gene-signature-score * MDM2.flowc |
| **5** | 4gscore.times.log2.MDM2.flowc | 4-gene-signature-score * log2(MDM2.flowc) |
| **6** | 4gscore.plus.log2.MDM2.flowc | 4-gene-signature-score + log2(MDM2.flowc) |
| gscore = gene signature score with either 3 or 4 genes according to Table 1, as disclosed in WO 2015/000945. MDM2.flowc = relative amount of MDM2 expressing cell type as measured by flow cytometry. | | |

The combined score "S" as obtainable according to the algorithms in Table 2 correlates to the patient's response to treatment with said MDM2-inhibitor. In one embodiment, a value for "S" above a standard value (or reference value) indicates the patient's likelihood to respond to or benefit from treatment with said MDM2-inhibitor, whereas a relative amount below said standard value indicates that said patient is less likely to respond to or benefit from that treatment. The standard value may depend on the algorithm used for calculating "S". For example, the reference level is different when a 3-gene or 4-gene signature is used in the calculation for S, or when the log2 scale is applied (see Fig. 4). The term "response" is as defined herein before. Likewise, the term "MDM2-inhibitor" is as defined above. Preferably such MDM2-inhibitor is a compound of formula IIa, or III, or (B), or (C), or (D) as defined herein, including any of the pharmaceutical preparation as defined herein before. In another embodiment, said MDM2-inhibitor can also be combined with other pharmaceutically active compounds, e.g. with cytarabine or cobimetinib, as also disclosed herein before.

In one embodiment, a 3- or 4-gene signature score is used in step c) of the above method. The 3-gene signature consists of mRNA expression levels of XPC, BBC3 and CDKN2A; or MDM2, XPC and BBC3; the 4-gene signature consists of mRNA expression levels of MDM2, XPC, BBC3 and CDKN2A. When mRNA expression levels are measured by RT-PCR, the patient's signature score is calculated from the sum of relative expression levels ($2^{delta\text{-}CP}$) measured at baseline (i.e. prior to treatment), multiplied by the observed direction *in-vitro,* defined as a 4-gene signature score: $S_{4\text{-gene-RT-1CR}} = G_{MDM2} + G_{XPC} + G_{BBC3} - G_{CDKN2A}$, where $G_x = 2^{delta\text{-}CPx}$ and delta-$CP_{MDM2} = CP_{housekeeper}\text{-}CP_{MDM2}$, delta-$CP_{XPC} = CP_{housekeeper}\text{-}CP_{XPC}$, delta-$CP_{BBC3} = CP_{housekeeper}\text{-}CP_{BBC3}$, delta-$CP_{CDKN2A} = CP_{housekeeper}\text{-}CP_{CDKN2A}$. The term "housekeeper" means any suitable control gene. In one embodiment of the present invention the "housekeeper" is TMEM55B as the endogenous control gene.

Let $S_{MDM2\text{-}flow}$ denote the percentage of cells having detectable MDM2 protein expression in a selected cell type, for example the CD45 dim blast cell population. By combining the protein expression measurement of MDM2 $S_{MDM2\text{-}flow}$ and the 4-gene signature score $S_{4\text{-gene-RT-PCR}}$ based on RT-PCR, the combined score "S" can be calculated in the following three ways:

$$S = S_{4\text{-gene-RT-PCR}} \times S_{MDM2\text{-flow}};$$

or

$$S = S_{4\text{-gene-RT-PCR}} \times \log2(S_{MDM2\text{-flow}});$$

or

$$S = S_{4\text{-gene-RT-PCR}} + \log2(S_{MDM2\text{-flow}}).$$

Similarly, given a 3-gene signature score $S_{3\text{-gene-RT-PCR}} = G_{XPC} + G_{BBC3} - G_{CDKN2A}$, which omits $G_{MDM2}$ from score calculation, the combined score "S" can be calculated in the same ways:

$$S = S_{3\text{-gene-RT-PCR}} \times S_{MDM2\text{-flow}};$$

or

$$S = S_{3\text{-gene-RT-PCR}} \times \log2(S_{MDM2\text{-flow}});$$

or

$$S = S_{3\text{-gene-RT-PCR}} + \log2(S_{MDM2\text{-flow}}).$$

Likewise, the above ways for calculating the combined score "S" can also be applied for a 3-gene signature score $S_{3\text{-gene-RT-PCR}} = G_{XPC} + G_{BBC3} + G_{MDM2}$, which omits $G_{CDKN2A}$ from score calculation.

The combined signature score "S" was correlated with clinical responses of the NP29679 trial (indication: AML) Part 1 and 2 by testing whether signature scores based on any of the above six definitions can significantly differentiate responders from non-responders using two-sample Wilcoxon rank sum tests and via computing classification area-under-the-curves (AUC).

Therefore, in one embodiment, step b) of the above method comprises detection of the relative amount of

i) MDM2 positive CD45dim cells; or
ii) MDM2 positive CD117+ (c-kit) and CD45dim cells; or
iii) MDM2 positive CD34+ and CD45dim cells; or
iv) MDM2 positive CD34+ and CD117+ and CD45dim cells; each of the values obtained under i) to iv) is designated as $S_{MDM2\text{-flow}}$; and

step c) comprises detection of mRNA levels of a 3-gene signature by RT-PCR, wherein the 3-gene signature comprises XPC, BBC3 and CDKN2A or MDM2, XPC and BBC3 and is designated as $S_{3\text{-gene}}$; and
the combined score "S" in step d) is calculated by multivariate analysis according to an algorithm selected from the group consisting of

$$S = S_{3\text{-gene}} \times S_{MDM2\text{-flow}};$$

or

$$S = S_{3\text{-gene}} \times \log2(S_{MDM2\text{-flow}});$$

or

$$S = S_{3\text{-gene}} + \log2(S_{MDM2\text{-flow}});$$

and steps a), e) and f) are as defined above.

In another embodiment, step b) of the above method comprises detection of the relative amount of

i) MDM2 positive CD45dim cells; or
ii) MDM2 positive CD117+ (c-kit) and CD45dim cells; or
iii) MDM2 positive CD34+ and CD45dim cells; or
iv) MDM2 positive CD34+ and CD117+ and CD45dim cells; each of the values obtained under i) to iv) is designated as $S_{MDM2\text{-flow}}$; and

step c) comprises detection of mRNA levels of a 4-gene signature by RT-PCR, wherein the 4-gene signature comprises MDM2, XPC, BBC3 and CDKN2A and is designated as $S_{4\text{-gene}}$; and the combined score "S" in step d) is calculated by multivariate analysis according to an algorithm selected from the group consisting of

$$S = S_{4\text{-gene}} \times S_{MDM2\text{-flow}};$$

or

$$S = S_{4\text{-gene}} \times \log2(S_{MDM2\text{-flow}});$$

or

$$S = S_{4\text{-gene}} + \log2(S_{MDM2\text{-flow}});$$

and

steps a), e) and f) are as defined above.

In yet another embodiment, there is provided an *in vitro* method to predict responsiveness of a patient, with cancer, to a therapy, said method comprises the following steps:

a) measuring the relative amount (%) of MDM2 protein expressing CD45dim (Blast) cells in blood and/or bone marrow biopsy sample(s) ($S_{MDM2\text{-flow}}$) obtained from the patient at baseline

b) measuring mRNA expression levels of MDM2, XPC, BBC3 and CDKN2A in blood and/or bone marrow biopsy sample(s) obtained from the patient at baseline by RT-PCR, and calculating a 3-gene signature score $S_{3\text{-gene-RT-PCR}}$ according to the formula

$$S_{3\text{-gene-RT-PCR}} = G_{XPC} + G_{BBC3} - G_{CDKN2A},$$

or

$$S_{3\text{-gene-RT-PCR}} = G_{XPC} + G_{BBC3} + G_{MDM2};$$

where $G_x = 2^{\text{delta-CPx}}$ and delta-$CP_x = CP_{housekeeper}$-$CP_x$ ;

c) applying a multivariate analysis using the values obtained in a) and b) according to one of the following algorythms, in order to calculate a value S

$$S = S_{3\text{-gene-RT-PCR}} \times S_{MDM2\text{-flow}};$$

or

$$S = S_{3\text{-gene-RT-PCR}} \times \log2(S_{MDM2\text{-flow}});$$

or

$$S = S_{3\text{-gene-RT-PCR}} + \log2(S_{MDM2\text{-flow}});$$

d) comparing said value for "S" obtained in c) to standard values, for example values obtained from a patient with the same cancer.

A compound which acts as inhibitor of the MDM2-p53 interaction as defined herein is administered when the value for "S" obtained in c) is above said standard value.

Within this embodiment, the "standard value" (or reference value) may differ according to the algorithm applied in step d). Therefore, in a preferred embodiment, when "S" is calculated according to $S = S_{3\text{-gene-RT-PCR}} \times S_{MDM2\text{-flow}}$ the standard value for comparison according to step e) is about 50, or about 100, or about 150 (see **Fig. 4b ii) and v)**). In another preferred embodiment, when "S" is calculated according to $S = S_{3\text{-gene-RT-PCR}} \times \log2(S_{MDM2\text{-flow}})$, the standard value for comparison according to step e) is about 8, or about 10, or about 12, or about 15 (see **Fig. 4b i) and iv)**). In another preferred embodiment, when "S" is calculated according to $S = S_{3\text{-gene-RT-PCR}} + \log2(S_{MDM2\text{-flow}})$, the standard value for comparison according to step e) is about 7, or about 8, or about 8.5, or about 9 (see **Fig. 4b iii) and vi)**).

In yet another embodiment, there is provided an *in vitro* method to predict responsiveness of a patient, with cancer, to a therapy, said method comprises the following steps:

a) measuring the relative amount ($S_{MDM2\text{-}flow}$ in %) of MDM2 protein expressing CD45dim (Blast) cells in blood and/or bone marrow biopsy sample(s) obtained from the patient at baseline
b) measuring mRNA expression levels of MDM2, XPC, BBC3 and CDKN2A in blood and/or bone marrow biopsy sample(s) obtained from the patient at baseline by RT-PCR, and calculating a 4-gene signature score $S_{4\text{-}gene\text{-}RT\text{-}PCR}$ according to the formula

$$S_{4\text{-}gene\text{-}RT\text{-}PCR} = G_{MDM2} + G_{XPC} + G_{BBC3} - G_{CDKN2A}$$

where $G_x = 2^{delta\text{-}CPx}$ and delta-$CP_x$ = $CP_{housekeeper}$-$CP_x$ ;
c) applying a multivariate analysis using the values obtained in a) and b) according to one of the following algorythms, in order to calculate a value S

$$S = S_{4\text{-}gene\text{-}RT\text{-}PCR} \times S_{MDM2\text{-}flow};$$

or

$$S = S_{4\text{-}gene\text{-}RT\text{-}PCR} \times \log2(S_{MDM2\text{-}flow});$$

or

$$S = S_{4\text{-}gene\text{-}RT\text{-}PCR} + \log2(S_{MDM2\text{-}flow});$$

d) comparing said value for "S" obtained in c) to standard values, for example values obtained from a patient with the same cancer.

A compound which acts as inhibitor of the MDM2-p53 interaction as defined herein is administered when the value for "S" obtained in d) is above said standard value.

Within this embodiment, the "standard value" (or reference value) may differ according to the algorithm applied in step d). Therefore, in a preferred embodiment, when "S" is calculated according to $S = S_{4\text{-}gene\text{-}RT\text{-}PCR} \times S_{MDM2\text{-}flow}$ the standard value for comparison according to step e) is about 200, or about 300, or about 400, or about 450, or about 500 (see **Fig. 4a i) and iv)**). In another preferred embodiment, when "S" is calculated according to $S = S_{4\text{-}gene\text{-}RT\text{-}PCR} \times \log2(S_{MDM2\text{-}flow})$, the standard value for comparison according to step e) is about 27, or about 30, or about 33, or about 36, or about 40 (see **Fig. 4a ii) and v)**). In another preferred embodiment, when "S" is calculated according to $S = S_{4\text{-}gene\text{-}RT\text{-}PCR} + \log2(S_{MDM2\text{-}flow})$, the standard value for comparison according to step e) is about 10, or about 10.5, or about 11, or about 11.5 or about 12 (see **Fig. 4a iii) and vi)**).

In another embodiment, the combined score "S" as defined in any of the embodiments above is used during treatment, i.e. monitoring of a cancer patient's treatment with an MDM2-inhibitor as defined herein before. Within this embodiment, the sample(s) in step a) according to any of the methods described above is taken during treatment, and the treatment is continued when the combined score obtained in d) is above the corresponding standard value.

In another embodiment, the present invention provides a method for determining a cancer patient's response to treatment with an MDM2 inhibitor as defined herein, characterized in that the higher the combined score "S", as obtained according to any algorithm defined herein before, the higher is the likelihood of said patient's response to said treatment as for example indicated by ROC (AUC) values and analyses, well known to the person of skill in the art of clinical pharmacology.

The combined score "S", as obtained according to any of the methods/algorithms defined above, can be used in the context of p53 gene mutation status to even more accurately discriminate patient responses/benefits to treatment. The patient's p53 gene mutation status may serve as an additional marker to detect said patients response to treatment with an MDM2 inhibitor as defined herein with improved sensitivity and/or specificity. Therefore, in yet another embodiment, the present invention provides any of the methods for obtaining the combined score "S" as defined herein, further comprising the step of detecting p53 gene mutation status in said patient's sample. Within this embodiment, the p53 gene mutation status is preferably wild-type. However, detection of p53 gene mutations should not in general be understood as an exclusion criterion for that patient's likelihood to respond to - or benefit from treatment with an MDM2-inhibitor as disclosed herein. Thus, in yet another embodiment the present invention provides a combined, multivariate analysis combining % MDM2 expressing cells as defined herein, in particular CD45dim (blast) cells with gene signature scores, preferably 3- and 4 gene signature scores as defined above, and p53 mutation status in order to provide a more refined prediction of said patient's likelihood to meet a beneficial clinical endpoint, i.e. to respond to treatment. In still another embodiment, further co-variates in this multivariate analysis are the patient's age and/or ECOG score.

Also disclosed herein is a method for treating a cancer patient comprising obtaining a combined score "S" according to any one of the methods defined above, and administering to said patient an MDM2-inhibitor if said value for "S" is above a standard - or reference value as also defined herein before. In one disclosure, the MDM2-inhibitor is as defined herein before. In another disclosure the MDM2-inhibitor is a compound of formula (II), (IIa), (B), (C) or (D) as defined above. In another disclosure, the p-53 mutation status as also detected in a sample obtained from said cancer patient is preferably wild-type.

In one aspect the disclosure relates to a kit and in another aspect to a device for predicting the response, preferably of a cancer in a subject (or patient), to a compound of general formula I, II, III; or IIa or III; or (A), (B), (C) or (D) or a pharmaceutically acceptable derivative thereof as defined herein, said kit comprising means, tools or devices for sample collection, especially for collection of blood- or bone marrow samples and instructions how to detect relative amounts of MDM2 protein expressing cells as defined herein, in particular CD45dim (blast) cells, and mRNA levels of one ore several genes, preferably a 3- or 4-gene signature, according to Table 1 in said samples.

The kit and device also comprises instructions how to obtain the combined score "S" from the detected releative amount(s) of MDM2-protein expressing cells and mRNA levels. The kit and device may also preferably comprise a comparator module which comprises a standard value or set of standard values to which the value of "S" obtained from the patient's sample(s) is compared.

In another disclosure, there is provided a kit for predicting the response to treatment with a medicament comprising a compound which acts as inhibitor of the MDM2-p53 interaction, comprising;

a) means for obtaining a sample, preferably a blood- or bone marrow sample from a patient suffering from cancer, in particular a hematological disorder such as leukemia, and in particular AML;
b) instructions how to detect

    i) the relative amount of one or several of MDM2 protein expressing cell types as defined herein, in particular CD45dim (blast) cells, from that sample, preferably by using flow cytometry,
    ii) mRNA expression levels of XPC, BBC3, CDKN2A and optionally MDM2, preferably by using RT-PCR; and
    iii) optionally also detecting co-variates selected from the patient's p53 gene mutation status, age and/or ECOG score;

c) a comparator module, comprising standard values and instructions how to use them; and
d) a medicament comprising a compound which acts as inhibitor of the MDM2-p53 interaction.

In yet another disclosure there is provided a kit as described above, wherein the compound acting as inhibitor of the MDM2-p53 interaction is a compound formula I, II, III; or IIa or III; or (A), (B), (C) or (D) or a pharmaceutically acceptable derivative thereof as defined herein. The standard value or set of standard values may be determined as described above.

The following examples are illustrative of the invention and not limitative thereof.

## EXAMPLES

### EXAMPLE 1

This Example describes the detection of the relative amount of MDM2 expressing CD45dim blast cells obtained from patients participating in Study NP28679 (Part 1 and 2) using flow cytometry.

*Material*

CD34 PerCP BD (Becton Dichinson) catalog number 340666
CD45 AF700 Custom manufactured by sponsored CRO
Mdm2 AF647 Santa Cruz Biotech, catalog number sc-5304AF647

Perm Buffer III BD catalog number 558050
BD Lyse/ Fix catalog number 558049
PBS with 1% BSA custom preparation according to SOP of sponsored CRO

*Staining procedure*

Cells were stained according to the following procedure: 100μl blood of appropriate donor was added to appropriately labelled tubes. Surface markers except CD34 were subsequently stained by adding antibody fluorochrome conjugates or respective isotype controls were used. Tubes were then vortexed and incubated for 20 minutes in the dark at room temperature. After washing with PBS BSA buffer, subes were centrifuged and decanted. 2 ml of prewarmed 1x BD Lyse/ fix solution was then added, tubes were vortexed and incubated 15 minutes in a 37°C water bath. After incubation, tubes were centrifuged and supernatant decanted. After two washing steps, the appropriate antibodies for intracellular staining or isotype controls were added with PBS/ BSA using the BD Lyse/Fix Perm buffer. CD34 or respective isotype was added. Tubes were incubated 30 minutes in the dark at room temperature. Tubes were then washed twice with PBS/ BSA and finally resuspended in 500 μl for acquisition in a FACSCanto II flow cytometer. 250'000 events were acquired.

*Electronic gating for result output*

For results generation, flow cytometry analysis software like Winlist or Flowjo must be used. Initially, a gate is drawn around all cells with normal size (forwards scatter, FSC) and granular characteristics (side scatter, SSC): the total nucleated cell gate (TNC, R1 in figure 1). Within this TNC subset, the AML blast population is then identified by drawing a region around cells with dim CD45 expression and low side scatter porperties (R3 in figure 1). The blast population can be further refined by gating on CD34 positive or negative blasts (Figure 1, R6 and R39, respectively). The threshold for CD34 positivity can be identified by using an isotype control staining in that respective channel (not shown).

Next, the CD45 dim general blast population and the more refined blast population additionally expressing CD34 are displayed in dot plots as shown in figure 2. The isotype control used instead of Mdm2 staining can be used to set the threshold for Mdm2 positivity (upper two panels figure 2.) The staining condition including Mdm2 recognizing antibody is displayed at the bottom of figure 2. The cell population falling in the regions defined as Mdm2 positive is determined by calculating the percentage of Mdm2 expressing cells within the respective blast parent population.

*Correlation of relative amount of MDM2 expressing CD45dim blast cells with patient response to treatment with MDM2-inhibitor of formula (B)*

The relative amount of MDM2 expressing CD45dim blast cells was obtained according to the above described method for patients participating in Study NP28679 (Part 1 and 2) and said amount plotted over the response of said patients (Fig. 3). Depending on whether partial response (PR) is included into the group of responders or not it is demonstrated that the relative amount of MDM2 expressing cells prior to treatment is indicative for that patient's response to treatment with the MDM2 inhibitor of formula (B). In particular, it is demonstrated that the higher the % MDM2 positve CD45 dim blast cells, among all CD45 blast cells detected in the patient's sample, the higher is the likelihood of improved clinical endpoint benefit ("response") for that patient, as for example indicated by ROC (AUC) values and analyses.

## EXAMPLE 2

This Example describes the calculation of the combined score "S" as defined herein, and its correlation with response of patients participating in Study NP28679 (Part 1 and 2). The relative amount of MDM2 positive CD45 dim cells were detected according to Example 1, and mRNA expression levels of MDM2, XPC, BBC3 and CDKN2A at baseline were measured by RT-PCR. Total RNA isolated from either blood or bone marrow is quantitated by UV absorption. For each PCR reaction a fixed total RNA input is used. PCR is performed using a proprietary DNA polymerase from Roche Molecular systems. The polymerase is capable of reverse transcription so that direct amplification and detection from RNA is performed in one reaction with a single set of gene specific primers. Real-time PCR utilizes TaqMan® technology with primers and TaqMan® probes for amplification and for detection of the target and control nucleic acids. Three reaction wells are used to detect the 4 target genes. In each reaction well the endogenous control is included. The cobas® z480 analyzer is used for amplification and detection.

The cycle-to-threshold value (Cp) is calculated for each fluorescent channel using onboard LightCycler®(LC) 480 software. Normalization of expression is done with TMEM55B as the endogenous control gene where the relative expression level (concentration) for a target gene (x) is defined as $Gx= 2^{delta\text{-}CPx}$ where $delta\text{-}Cp_x = Cp\ (TMEM) - Cp_x$, and target genes (x) are MDM2, BBC3, XPC and CDKN2A.

The patient's signature score was then calculated from the sum of relative expression levels ($G_x$) measured at baseline (i.e. prior to treatment), multiplied by the observed direction in-vitro, defined as a 4-gene signature score $S_{4\text{-gene-RT-PCR}} = G_{MDM2} + G_{XPC} + G_{BBC3} - G_{CDKN2A}$. Likewise, the 3-gene signature score is defined as $S_{3\text{-gene-RT-PCR}} = G_{XPC} + G_{BBC3} - G_{CDKN2A}$.

For the subsequent calculation of the combined score "S", let $S_{MDM2\text{-flow}}$ denote the percentage of cells having detectable MDM2 protein expression in the CD45 dim blast population. By combining the protein expression measurement of MDM2 $S_{MDM2\text{-flow}}$ and the 4-gene signature score $S_{4\text{-gene-RT-PCR}}$ based on RT-PCR, the combined score "S" was calculated in the following three ways:

1.

$$S = S_{4\text{-gene-RT-PCR}} \times S_{MDM2\text{-flow}};$$

2.

$$S = S_{4\text{-gene-RT-PCR}} \times \log2(S_{MDM2\text{-flow}});$$

3.

$$S = S_{4\text{-gene-RT-PCR}} + \log2(S_{MDM2\text{-flow}}).$$

Similarly, given a 3-gene signature score $S_{3\text{-gene-RT-PCR}} = G_{XPC} + G_{BBC3} - G_{CDKN2A}$, which omits $G_{MDM2}$ from score calculation, "S" was calculated in the same ways:

1.

$$S = S_{3\text{-gene-RT-PCR}} \times S_{MDM2\text{-flow}};$$

2.

$$S = S_{3\text{-gene-RT-PCR}} \times \log2(S_{MDM2\text{-flow}});$$

3.

$$S = S_{3\text{-gene-RT-PCR}} + \log2(S_{MDM2\text{-flow}}).$$

The combined signature score "S" was then correlated with clinical responses seen in clinical trials with patients suffering from AML (NP29679 trial Part 1 and 2) by testing whether signature scores based on any of the above six definitions can significantly differentiate responders from non-responders using two-sample Wilcoxon rank sum tests and via computing classification area-under-the-curves (AUC). The results are shown in Fig. 4a and 4b. Comparative data correlating the patient's response with mRNA expression levels of each of MDM2, BBC3, XPC and CDKN2A *alone* are shown in Fig. 5.

**Claims**

1. An *in vitro* method to predict responsiveness of a patient, with cancer, to a therapy, said method comprising detecting the relative amount of one or several MDM2 protein expressing cell types in a sample obtained from said cancer patient prior to treatment and using said relative amount, or percentage (%), of cells expressing MDM2 protein as a biomarker for predicting said patient's response to treatment with a medicament, wherein the medicament comprises a compound acting as inhibitor of the MDM2-p53 interaction, and wherein said MDM2 protein expressing cell type, or biomarker, is selected from the group consisting of CD45dim cells, CD117+ (c-kit) or CD34+ stem cells, or

combinational subsets thereof, and wherein said inhibitor of the MDM2-p53 interaction is selected from the compounds: 4-{[2R,3S,4R,5S)-4-(4-Cholor-2-fluoro-phenyl)-3-(3-chloro-2-fluoro-phenyl)-4-cyano-5-(2,2-dimethyl-propyl)-pyrrolidine-2-carbonyl]-amino}-3-methoxy-benzoic acid of the formula (B)

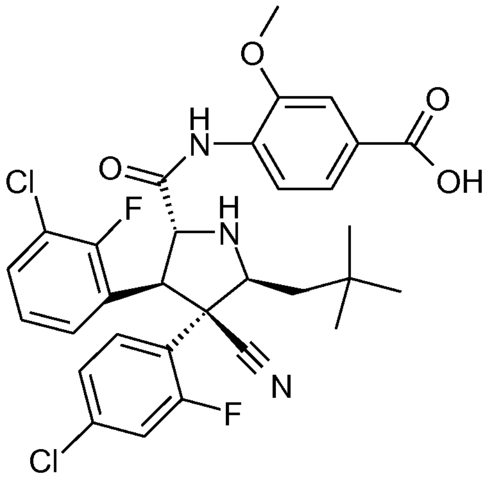

(B)

and 4-{[(2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carbonyl]-amino}-3-methoxy-benzoic acid 1-mPEG-carbonyloxy-ethyl ester (mPEG, average MW, ~2000) of the formula (C)

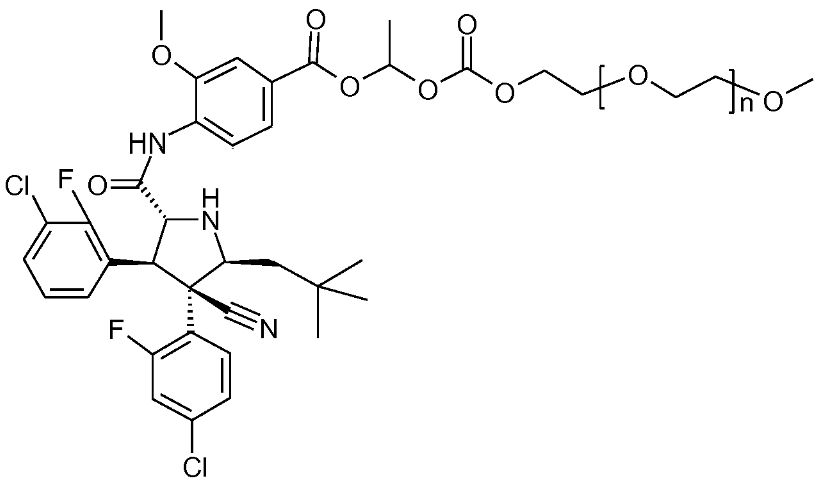

(C).

2. The method according to claim 1, said method comprising:

a) detecting said relative amount of MDM2 protein expressing CD45dim blast cells in a sample obtained from a patient suffering from cancer;
b) comparing the relative amount of MDM2 protein expressing CD45dim blast cells obtained in a) to standard values from a patient with the same cancer.

3. The method according to claim 1 or 2, wherein the cancer is selected from the group consisting of breast cancer, prostate cancer, cervical cancer, ovarian cancer, gastric cancer, colorectal cancer, pancreatic cancer, liver cancer, brain cancer, neuroendocrine cancer, lung cancer, kidney cancer, hematological malignancies, melanoma and sarcomas.

4. The method according to claim 3, wherein said patient suffers from a hematological cancer, in particular leukemia.

5. The method according to claim 4, wherein said patient suffers from acute myeloid leukemia.

**6.** The method according to any one of claims 1 to 5, wherein the relative amount of MDM2 protein expressing CD45dim blast cells is detected by flow cytometry.

**7.** The method according to any one of claim 1 or 7, wherein the sample obtained from said patient is a blood sample or a bone marrow sample.

**8.** The method according to any one of claims 1 to 7, wherein the relative amount of MDM2 protein expressing CD45dim blast cells detected in said sample obtained from said patient prior to treatment exceeds 45% or 55%.

**9.** The method according to claim 1, said method comprising:

a) detecting the relative amount of MDM2 protein expressing CD45dim blast cells in a blood- or bone marrow sample obtained from a patient suffering from acute myeloid leukemia by flow cytometry;
b) comparing the relative amount of MDM2 protein expressing CD45dim blast cells obtained in a) to standard values from a patient with the same cancer.

**10.** The method according to any one of claims 1 to 9, further comprising the step of detecting the cancer patient's p53 gene mutation status, prior to treatment.

**11.** The *in vitro* use of MDM2 protein expressing CD45dim blast cells for predicting the response of a patient suffering from cancer to treatment with a medicament comprising a compound which acts as inhibitor of the MDM2-p53 interaction, and wherein said compound which acts as inhibitor of the MDM2-p53 interaction is a compound of formula (B) or (C) according to claim 1.

**12.** The *in vitro* use according to claim 11, wherein the patient suffers from a hematological disorder, such as leukemia, in particular acute myeloid leukemia.

**13.** The method according to any one of claims 1 to 10, further comprising detecting a mRNA expression level of at least one gene selected from the group consisting of BAX, RPS27L, EDA2R, XPC, DDB2, FDXR, MDM2, CDKN1A, TRIAP1, BBC3, CCNG1, TNFRSF10B, or CDKN2A and combining that value with the relative amount of MDM2-protein expressing cells according to claim 1 in order to obtain a combined score "S", and using that combined score "S" as a biomarker for predicting the patient's response to a compound, wherein the compound is an inhibitor of the MDM2-p53 interaction according to claim 1.

**14.** The method of claim 13, wherein said method is an *in vitro* method to predict responsiveness of a patient, with cancer, to a therapy comprising the following steps:

a) measuring the relative amount (%) of MDM2 protein expressing CD45dim (Blast) cells in a blood and/or bone marrow biopsy sample ($S_{MDM2\text{-}flow}$) obtained from a patient suffering from acute myeloid leukemia at baseline;
b) measuring mRNA expression levels of MDM2, XPC, BBC3 and CDKN2A in a sample obtained in a) by RT-PCR, and calculating a 3-gene signature score $S_{3\text{-}gene\text{-}RT\text{-}PCR}$ according to the formula

$$S_{3\text{-gene-RT-PCR}} = G_{XPC} + G_{BBC3} - G_{CDKN2A}$$

or

$$S_{3\text{-gene-RT-PCR}} = G_{XPC} + G_{BBC3} + G_{MDM2};$$

where $G_x = 2^{delta\text{-}CPx}$ and delta-$CP_x$ = $CP_{housekeeper}$-$CP_x$ ;
c) applying a multivariate analysis using the values obtained in a) and b) according to one of the following algorythms, in order to calculate a value S

$$S = S_{3\text{-gene-RT-PCR}} \times S_{MDM2\text{-flow}};$$

or

$$S = S_{3\text{-gene-RT-PCR}} \times \log2(S_{MDM2\text{-flow}});$$

or

$$S = S_{3\text{-gene-RT-PCR}} + \log2(S_{MDM2\text{-flow}});$$

d) comparing said value for "S" obtained in c) to standard values, for example values obtained from a patient with the same cancer.

**15.** The method of claim 13, wherein said method is an *in vitro* method to predict responsiveness of a patient, with cancer, to a therapy comprising the following steps:

a) measuring the relative amount ($S_{MDM2\text{-flow}}$ in %) of MDM2 protein expressing CD45dim (Blast) cells in a blood and/or bone marrow biopsy sample obtained from a patient suffering from AML at baseline;
b) measuring mRNA expression levels of MDM2, XPC, BBC3 and CDKN2A in a sample obtained in a) by RT-PCR, and calculating a 4-gene signature score $S_{4\text{-gene-RT-PCR}}$ according to the formula

$$S_{4\text{-gene-RT-PCR}} = G_{MDM2} + G_{XPC} + G_{BBC3} - G_{CDKN2A}$$

where $G_x = 2^{\text{delta-CPx}}$ and delta-$CP_x = CP_{housekeeper}$-$CP_x$ ;
c) applying a multivariate analysis using the values obtained in a) and b) according to one of the following algorythms, in order to calculate a value S

$$S = S_{4\text{-gene-RT-PCR}} \times S_{MDM2\text{-flow}};$$

or

$$S = S_{4\text{-gene-RT-PCR}} \times \log2(S_{MDM2\text{-flow}});$$

or

$$S = S_{4\text{-gene-RT-PCR}} + \log2(S_{MDM2\text{-flow}});$$

d) comparing said value for "S" obtained in c) to standard values, for example values obtained from a patient with the same cancer.

**16.** A method according to claims 1, 14 or 15 further comprising at least one additional co-variate independently selected from the group of the patient’s p53 mutation status, age or ECOG score.

**Patentansprüche**

**1.** *In-vitro*-Verfahren zum Vorhersagen des Ansprechvermögens eines Patienten mit Krebs auf eine Therapie, wobei das Verfahren das Nachweisen der relativen Menge von einem oder mehreren MDM2-Protein exprimierenden Zelltypen in einer Probe, die vor der Behandlung von dem Krebspatienten erhalten wurde, und das Verwenden der relativen Menge, oder des Prozentsatzes (%), von Zellen, die MDM2-Protein exprimieren, als ein Biomarker zum Vorhersagen des Ansprechens des Patienten auf die Behandlung mit einem Medikament umfasst, wobei das Medikament eine Verbindung umfasst, die als ein Inhibitor der MDM2-p53-Wechselwirkung agiert, und wobei der MDM2-Protein exprimierende Zelltyp, oder der Biomarker, ausgewählt ist aus der Gruppe, bestehend aus CD45dim-Zellen, CD117+ (c-kit) oder CD34+ Stammzellen oder Kombinationsteilmengen davon, und wobei der Inhibitor der MDM2-p53-Wechselwirkung aus den folgenden Verbindungen ausgewählt ist: 4-{[2R,3S,4R,5S)-4-(4-Chlor-2-fluor-phenyl)-3-(3-chlor-2-fluor-phenyl)-4-cyano-5-(2,2-dimethyl-propyl)-pyrrolidin-2-carbonyl]-amino}-3-methoxy-ben-

zoesäure der Formel (B)

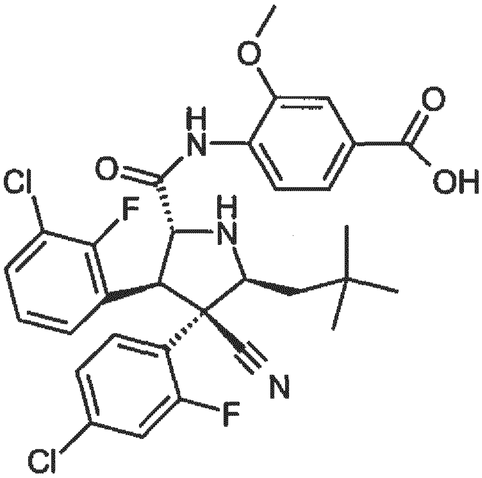

(B)

und 4-{[(2R,3S,4R,5S)-3-(3-Chlor-2-fluor-phenyl)-4-(4-chlor-2-fluor-phenyl)-4-cyano-5-(2,2-dimethyl-propyl)-pyrrolidin-2-carbonyl]-amino}-3-methoxy-benzoesäure-1-mPEG-carbonyloxy-ethylester (mPEG, mittleres MG, ~2000) der Formel (C)

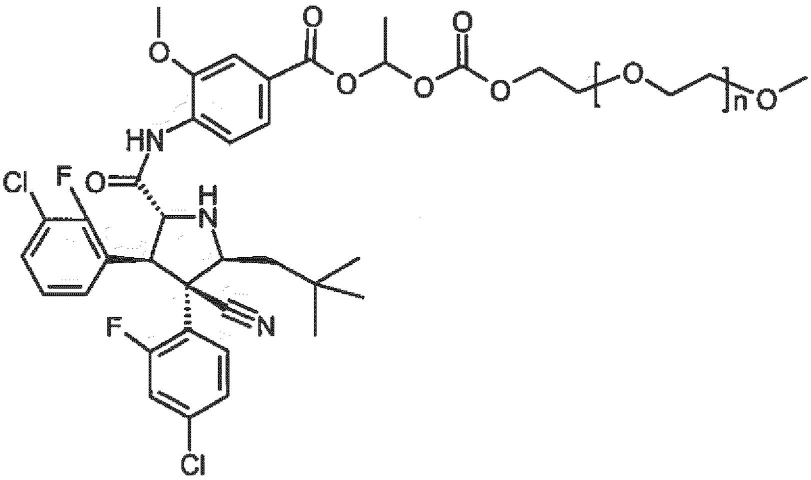

(C).

2. Verfahren nach Anspruch 1, wobei das Verfahren Folgendes umfasst:

   a) Nachweisen der relativen Menge von MDM2-Protein exprimierenden CD45dim-Blastzellen in einer Probe, die von einem Patienten erhalten wurde, der an Krebs leidet;
   b) Vergleichen der relativen Menge von MDM2-Protein exprimierenden CD45dim-Blastzellen, die in a) erhalten wurde, mit Standardwerten von einem Patienten mit dem gleichen Krebs.

3. Verfahren nach Anspruch 1 oder 2, wobei der Krebs ausgewählt ist aus der Gruppe, bestehend aus Brustkrebs, Prostatakrebs, Gebärmutterhalskrebs, Eierstockkrebs, Magenkrebs, Dickdarmkrebs, Bauchspeicheldrüsenkrebs, Leberkrebs, Hirnkrebs, neuroendokrinem Krebs, Lungenkrebs, Nierenkrebs, hämatologischen Malignitäten, Melanom und Sarkomen.

4. Verfahren nach Anspruch 3, wobei der Patient an einem hämatologischen Krebs, insbesondere Leukämie leidet.

5. Verfahren nach Anspruch 4, wobei der Patient an akuter myeloischer Leukämie leidet.

6. Verfahren nach einem der Ansprüche 1 bis 5, wobei die relative Menge von MDM2-Protein exprimierenden CD45dim-Blastzellen mit Hilfe von Durchflusszytometrie nachgewiesen wird.

**7.** Verfahren nach einem von Anspruch 1 oder 7, wobei die von dem Patienten erhaltene Probe eine Blutprobe oder eine Knochenmarkprobe ist.

**8.** Verfahren nach einem der Ansprüche 1 bis 7, wobei die relative Menge von MDM2-Protein exprimierenden CD45dim-Blastzellen, die in der von dem Patienten vor der Behandlung erhaltenen Probe nachgewiesen wurde, 45 % oder 55 % übersteigt.

**9.** Verfahren nach Anspruch 1, wobei das Verfahren Folgendes umfasst:

a) Nachweisen der relativen Menge von MDM2-Protein exprimierenden CD45dim-Blastzellen in einer Blut- oder Knochenmarkprobe, die von einem Patienten erhalten wurde, der an akuter myeloischer Leukämie leidet, mit Hilfe von Durchflusszytometrie;
b) Vergleichen der relativen Menge von MDM2-Protein exprimierenden CD45dim-Blastzellen, die in a) erhalten wurde, mit Standardwerten von einem Patienten mit dem gleichen Krebs.

**10.** Verfahren nach einem der Ansprüche 1 bis 9, ferner umfassend den Schritt des Nachweisens des p53-Genmutationsstatus des Krebspatienten vor der Behandlung.

**11.** *In-vitro*-Verwendung von MDM2-Protein exprimierenden CD45dim-Blastzellen zum Vorhersagen des Ansprechens eines Patienten, der an Krebs leidet, auf eine Behandlung mit einem Medikament, das eine Verbindung umfasst, die als Inhibitor der MDM2-p53-Wechselwirkung agiert, und wobei die Verbindung, die als Inhibitor der MDM2-p53-Wechselwirkung agiert, eine Verbindung der Formel (B) oder (C) nach Anspruch 1 ist.

**12.** *In-vitro*-Verwendung nach Anspruch 11, wobei der Patient an einer hämatologischen Störung wie Leukämie, insbesondere akuter myeloischer Leukämie leidet.

**13.** Verfahren nach einem der Ansprüche 1 bis 10, ferner umfassend das Nachweisen eines mRNA-Expressionsniveaus von mindestens einem Gen, das ausgewählt ist aus der Gruppe, bestehend aus BAX, RPS27L, EDA2R, XPC, DDB2, FDXR, MDM2, CDKN1A, TRIAP1, BBC3, CCNG1, TNFRSF10B oder CDKN2A, und das Kombinieren dieses Wertes mit der relativen Menge von MDM2-Protein exprimierenden Zellen nach Anspruch 1 zum Erhalten einer kombinierten Wertung "S" und das Verwenden dieser kombinierten Wertung "S" als einen Biomarker zum Vorhersagen des Ansprechens des Patienten auf eine Verbindung, wobei die Verbindung ein Inhibitor der MDM2-p53-Wechselwirkung nach Anspruch 1 ist.

**14.** Verfahren nach Anspruch 13, wobei das Verfahren ein *In-vitro*-Verfahren zum Vorhersagen des Ansprechvermögens eines Patienten mit Krebs auf eine Therapie ist, umfassend die folgenden Schritte:

a) Messen der relativen Menge (%) von MDM2-Protein exprimierenden CD45dim-(Blast-)-Zellen in einer Blut- und/oder Knochenmarkbiopsieprobe ($S_{MDM2\text{-}Fluss}$), die von einem Patienten erhalten wurde, der an akuter myeloischer Leukämie leidet, an der Grundlinie;
b) Messen der mRNA-Expressionsniveaus von MDM2, XPC, BBC3 und CDKN2A in einer Probe, die in a) erhalten wurde, mit Hilfe von RT-PCR und Berechnen einer 3-Gensignatur-Wertung $S_{3\text{-}Gen\text{-}RT\text{-}PCR}$ gemäß der Formel

$$S_{3\text{-}Gen\text{-}RT\text{-}PCR} = G_{XPC} + G_{BBC3} - G_{CDKN2A}$$

oder

$$S_{3\text{-}Gen\text{-}RT\text{-}PCR} = G_{XPC} + G_{BBC3} + G_{MDM2};$$

wobei $G_X = 2^{delta\text{-}CPx}$ und $delta\text{-}CP_X = CP_{konstitutiv}\text{-}CP_X$;
c) Anwenden einer multivariaten Analyse unter Verwendung der in a) und b) erhaltenen Werte gemäß einem der folgenden Algorithmen zur Berechnung eines Wertes S

$$S = S_{3\text{-}Gen\text{-}RT\text{-}PCR} \times S_{MDM2\text{-}Fluss};$$

oder

$$S = S_{3\text{-Gen-RT-PCR}} \times \log2(S_{MDM2\text{-Fluss}});$$

oder

$$S = S_{3\text{-Gen-RT-PCR}} + \log2(S_{MDM2\text{-Fluss}});$$

d) Vergleichen des in c) erhaltenen Wertes für "S" mit Standardwerten, zum Beispiel Werten, die von einem Patienten mit dem gleichen Krebs erhalten wurden.

**15.** Verfahren nach Anspruch 13, wobei das Verfahren ein *In-vitro*-Verfahren zum Vorhersagen des Ansprechvermögens eines Patienten mit Krebs auf eine Therapie ist, umfassend die folgenden Schritte:

a) Messen der relativen Menge ($S_{MDM2\text{-Fluss}}$ in %) von MDM2-Protein exprimierenden CD45dim-(Blast-)-Zellen in einer Blut- und/oder Knochenmarkbiopsieprobe, die von einem Patienten erhalten wurde, der an AML leidet, an der Grundlinie;
b) Messen der mRNA-Expressionsniveaus von MDM2, XPC, BBC3 und CDKN2A in einer Probe, die in a) erhalten wurde, mit Hilfe von RT-PCR und Berechnen einer 4-Gensignatur-Wertung $S_{4\text{-Gen-RT-PCR}}$ gemäß der Formel

$$S_{4\text{-Gen-RT-PCR}} = G_{MDM2} + G_{XPC} + G_{BBC3} - G_{CDKN2A},$$

wobei $G_X = 2^{\text{delta-CPx}}$ und delta-$CP_X$ = $CP_{konstitutiv}$-$CP_X$;
c) Anwenden einer multivariaten Analyse unter Verwendung der in a) und b) erhaltenen Werte gemäß einem der folgenden Algorithmen zur Berechnung eines Wertes S

$$S = S_{4\text{-Gen-RT-PCR}} \times S_{MDM2\text{-Fluss}};$$

oder

$$S = S_{4\text{-Gen-RT-PCR}} \times \log2(S_{MDM2\text{-Fluss}});$$

oder

$$S = S_{4\text{-Gen-RT-PCR}} + \log2(S_{MDM2\text{-Fluss}});$$

d) Vergleichen des in c) erhaltenen Wertes für "S" mit Standardwerten, zum Beispiel Werten, die von einem Patienten mit dem gleichen Krebs erhalten wurden.

**16.** Verfahren nach den Ansprüchen 1, 14 oder 15, ferner umfassend mindestens eine zusätzliche Kovariate, unabhängig voneinander ausgewählt aus der Gruppe des p53-Mutationsstatus, dem Alter oder der ECOG-Wertung des Patienten.

## Revendications

**1.** Procédé *in vitro* pour prédire la réponse d’un patient, atteint d’un cancer, à une thérapie, ledit procédé comprenant la détection de la quantité relative d’un ou de plusieurs types de cellules exprimant la protéine MDM2 dans un échantillon obtenu auprès dudit patient atteint d’un cancer avant traitement et l’utilisation de ladite quantité relative, ou pourcentage (%), de cellules exprimant la protéine MDM2 en tant que biomarqueur pour prédire la réponse dudit patient à un traitement avec un médicament, dans lequel le médicament comprend un composé agissant comme

inhibiteur de l'interaction MDM2-p53, et dans lequel ledit type de cellule exprimant la protéine MDM2, ou biomarqueur, est choisi dans le groupe constitué des cellules CD45dim, des cellules souches CD117+ (c-kit) ou CD34+, ou de sous-ensembles combinatoires de celles-ci, et dans lequel ledit inhibiteur de l'interaction MDM2-p53 est choisi parmi les composés :

acide 4-{[2R,3S,4R,5S)-4-(4-chloro-2-fluoro-phényl)-3-(3-chloro-2-fluoro-phényl)-4-cyano-5-(2,2-diméthyl-propyl)-pyrrolidine-2-carbonyl]-amino}-3-méthoxy-benzoïque de formule (B)

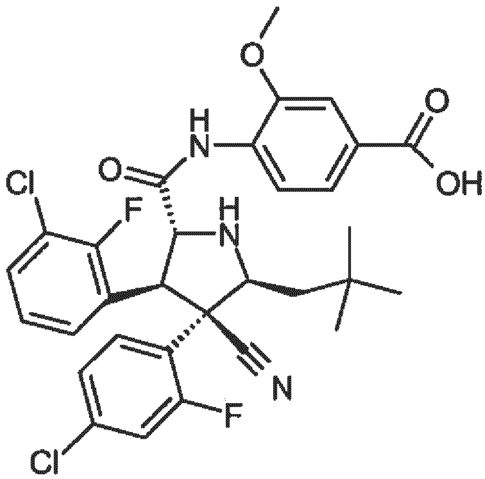

(B)

et

ester de 1-mPEG-carbonyloxy-éthyle de l'acide 4-{[(2R,3S,4R,5S)-3-(3-chloro-2-fluoro-phényl)-4-(4-chloro-2-fluoro-phényl)-4-cyano-5-(2,2-diméthyl-propyl)-pyrrolidine-2-carbonyl]-amino}-3-méthoxy-benzoïque (mPEG, MM moyenne, ~2000) de formule (C)

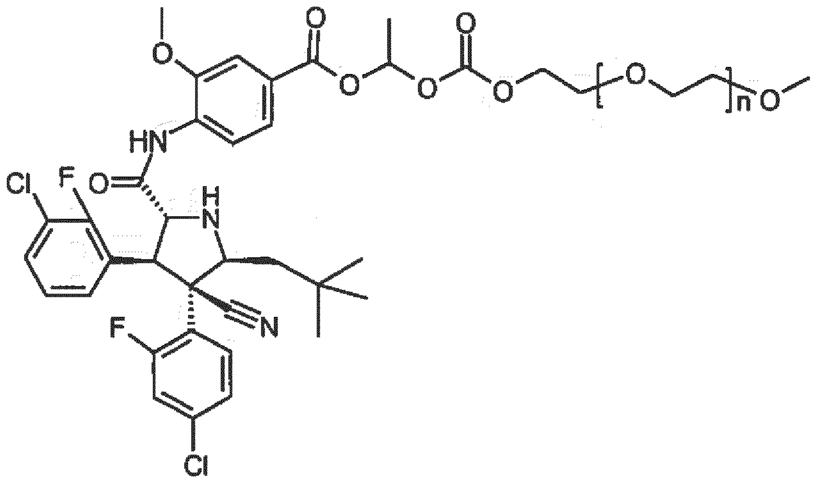

(C).

2. Procédé selon la revendication 1, ledit procédé comprenant :

a) la détection de ladite quantité relative de cellules blastiques CD45dim exprimant la protéine MDM2 dans un échantillon obtenu d'un patient souffrant d'un cancer ;
b) la comparaison de la quantité relative de cellules blastiques CD45dim exprimant la protéine MDM2 obtenue en a) à des valeurs de référence d'un patient atteint du même cancer.

3. Procédé selon la revendication 1 ou 2, dans lequel le cancer est choisi dans le groupe constitué du cancer du sein, du cancer de la prostate, du cancer du col de l'utérus, du cancer de l'ovaire, du cancer de l'estomac, du cancer colorectal, du cancer du pancréas, du cancer du foie, du cancer du cerveau, du cancer neuroendocrinien, du cancer du poumon, du cancer du rein, des malignités hématologiques, du mélanome et des sarcomes.

**4.** Procédé selon la revendication 3, dans lequel ledit patient souffre d'un cancer hématologique, en particulier d'une leucémie.

**5.** Procédé selon la revendication 4, dans lequel ledit patient souffre d'une leucémie myéloïde aiguë.

**6.** Procédé selon l'une quelconque des revendications 1 à 5, dans lequel la quantité relative de cellules blastiques CD45dim exprimant la protéine MDM2 est détectée par cytométrie en flux.

**7.** Procédé selon l'une quelconque de la revendication 1 ou 7, dans lequel l'échantillon obtenu dudit patient est un échantillon de sang ou un échantillon de moelle osseuse.

**8.** Procédé selon l'une quelconque des revendications 1 à 7, dans lequel la quantité relative de cellules blastiques CD45dim exprimant la protéine MDM2 détectée dans ledit échantillon obtenu dudit patient avant traitement dépasse 45 % ou 55 %.

**9.** Procédé selon la revendication 1, ledit procédé comprenant :

a) la détection de la quantité relative de cellules blastiques CD45dim exprimant la protéine MDM2 dans un échantillon de sang ou de moelle osseuse obtenu d'un patient souffrant d'une leucémie myéloïde aiguë par cytométrie en flux ;
b) la comparaison de la quantité relative de cellules blastiques CD45dim exprimant la protéine MDM2 obtenue en a) à des valeurs de référence d'un patient atteint du même cancer.

**10.** Procédé selon l'une quelconque des revendications 1 à 9, comprenant en outre l'étape de détection de l'état de mutation du gène p53 du patient atteint d'un cancer, avant traitement.

**11.** Utilisation *in vitro* de cellules blastiques CD45dim exprimant la protéine MDM2 pour prédire la réponse d'un patient souffrant d'un cancer à un traitement avec un médicament comprenant un composé qui agit comme inhibiteur de l'interaction MDM2-p53, et dans lequel ledit composé qui agit comme inhibiteur de l'interaction MDM2-p53 est un composé de formule (B) ou (C) selon la revendication 1.

**12.** Utilisation *in vitro* selon la revendication 11, dans laquelle le patient souffre d'un trouble hématologique, tel qu'une leucémie, en particulier une leucémie myéloïde aiguë.

**13.** Procédé selon l'une quelconque des revendications 1 à 10, comprenant en outre la détection d'un niveau d'expression d'ARNm d'au moins un gène choisi dans le groupe constitué de BAX, RPS27L, EDA2R, XPC, DDB2, FDXR, MDM2, CDKN1A, TRIAP1, BBC3, CCNG1, TNFRSF10B ou CDKN2A et la combinaison de cette valeur à la quantité relative de cellules exprimant la protéine MDM2 selon la revendication 1 afin d'obtenir un score combiné « S », et l'utilisation de ce score combiné « S » en tant que biomarqueur pour prédire la réponse du patient à un composé, dans lequel le composé est un inhibiteur de l'interaction MDM2-p53 selon la revendication 1.

**14.** Procédé selon la revendication 13, dans lequel ledit procédé est un procédé *in vitro* pour prédire la réponse d'un patient, atteint d'un cancer, à une thérapie comprenant les étapes suivantes :

a) la mesure de la quantité relative (%) de cellules (blastiques) CD45dim exprimant la protéine MDM2 dans un échantillon de biopsie de sang et/ou de moelle osseuse ($S_{MDM2\text{-}flux}$) obtenu d'un patient souffrant d'une leucémie myéloïde aiguë à la ligne de base ;
b) la mesure des niveaux d'expression d'ARNm de MDM2, XPC, BBC3 et CDKN2A dans un échantillon obtenu en a) par RT-PCR, et le calcul d'un score de signature de 3 gènes $S_{3\text{-gènes-RT-PCR}}$ selon la formule

$$S_{3\text{-gènes-RT-PCR}} = G_{XPC} + G_{BBC3} - G_{CDKN2A}$$

ou

$$S_{3\text{-gènes-RT-PCR}} = G_{XPC} + G_{BBC3} + G_{MDM2} ;$$

où $G_x = 2^{delta\text{-}CPx}$ et $delta\text{-}CP_x = CP_{domestique} - CP_x$ ;
c) l'application d'une analyse à plusieurs variables en utilisant les valeurs obtenues en a) et b) selon l'un des algorithmes suivants, pour calculer une valeur S

$$S = S_{3\text{-gènes-RT-PCR}} \times S_{MDM2\text{-flux}} ;$$

ou

$$S = S_{3\text{-gènes-RT-PCR}} \times \log2(S_{MDM2\text{-flux}}) ;$$

ou

$$S = S_{3\text{-gènes-RT-PCR}} + \log2(S_{MDM2\text{-flux}}) ;$$

d) la comparaison de ladite valeur pour « S » obtenue en c) à des valeurs de référence, par exemple à des valeurs obtenues d'un patient atteint du même cancer.

**15.** Procédé selon la revendication 13, dans lequel ledit procédé est un procédé *in vitro* pour prédire la réponse d'un patient, atteint d'un cancer, à une thérapie comprenant les étapes suivantes :

a) la mesure de la quantité relative ($S_{MDM2\text{-flux}}$ en %) de cellules (blastiques) CD45dim exprimant la protéine MDM2 dans un échantillon de biopsie de sang et/ou de moelle osseuse obtenu d'un patient souffrant d'une LMA à la ligne de base ;
b) la mesure des niveaux d'expression d'ARNm de MDM2, XPC, BBC3 et CDKN2A dans un échantillon obtenu en a) par RT-PCR, et le calcul d'un score de signature de 4 gènes $S_{4\text{-gènes-RT-PCR}}$ selon la formule

$$S_{4\text{-gènes-RT-PCR}} = G_{MDM2} + G_{XPC} + G_{BBC3} - G_{CDKN2A}$$

où $G_x = 2^{delta\text{-}CPx}$ et $delta\text{-}CP_x = CP_{domestique} - CP_x$ ;
c) l'application d'une analyse à plusieurs variables en utilisant les valeurs obtenues en a) et b) selon l'un des algorithmes suivants, pour calculer une valeur S

$$S = S_{4\text{-gènes-RT-PCR}} \times S_{MDM2\text{-flux}} ;$$

ou

$$S = S_{4\text{-gènes-RT-PCR}} \times \log2(S_{MDM2\text{-flux}}) ;$$

ou

$$S = S_{4\text{-gènes-RT-PCR}} + \log2(S_{MDM2\text{-flux}}) ;$$

d) la comparaison de ladite valeur pour « S » obtenue en c) à des valeurs de référence, par exemple à des valeurs obtenues d'un patient atteint du même cancer.

**16.** Procédé selon les revendications 1, 14 ou 15 comprenant en outre au moins une co-variable supplémentaire indépendamment choisie dans le groupe de l'état de mutation de p53, de l'âge ou du score ECOG du patient.

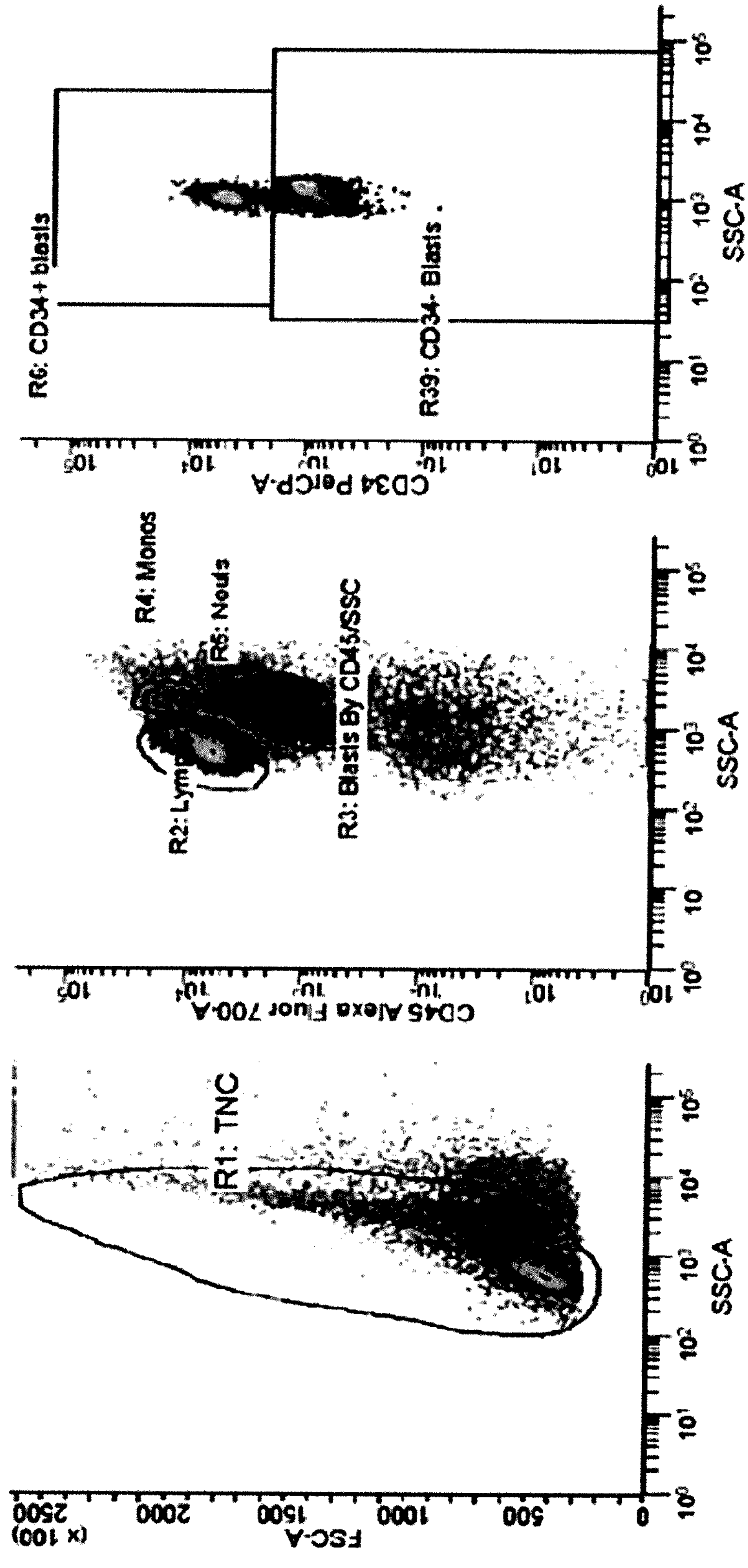

R1: TNC
FSC-A
(x 100)
SSC-A
CD45 Alexa Fluor 700-A
R2: Lymp
R3: Blasts By CD45/SSC
R4: Monos
R5: Neuts
CD34 PerCP-A
R6: CD34+ blasts
R39: CD34- Blasts


Fig. 1

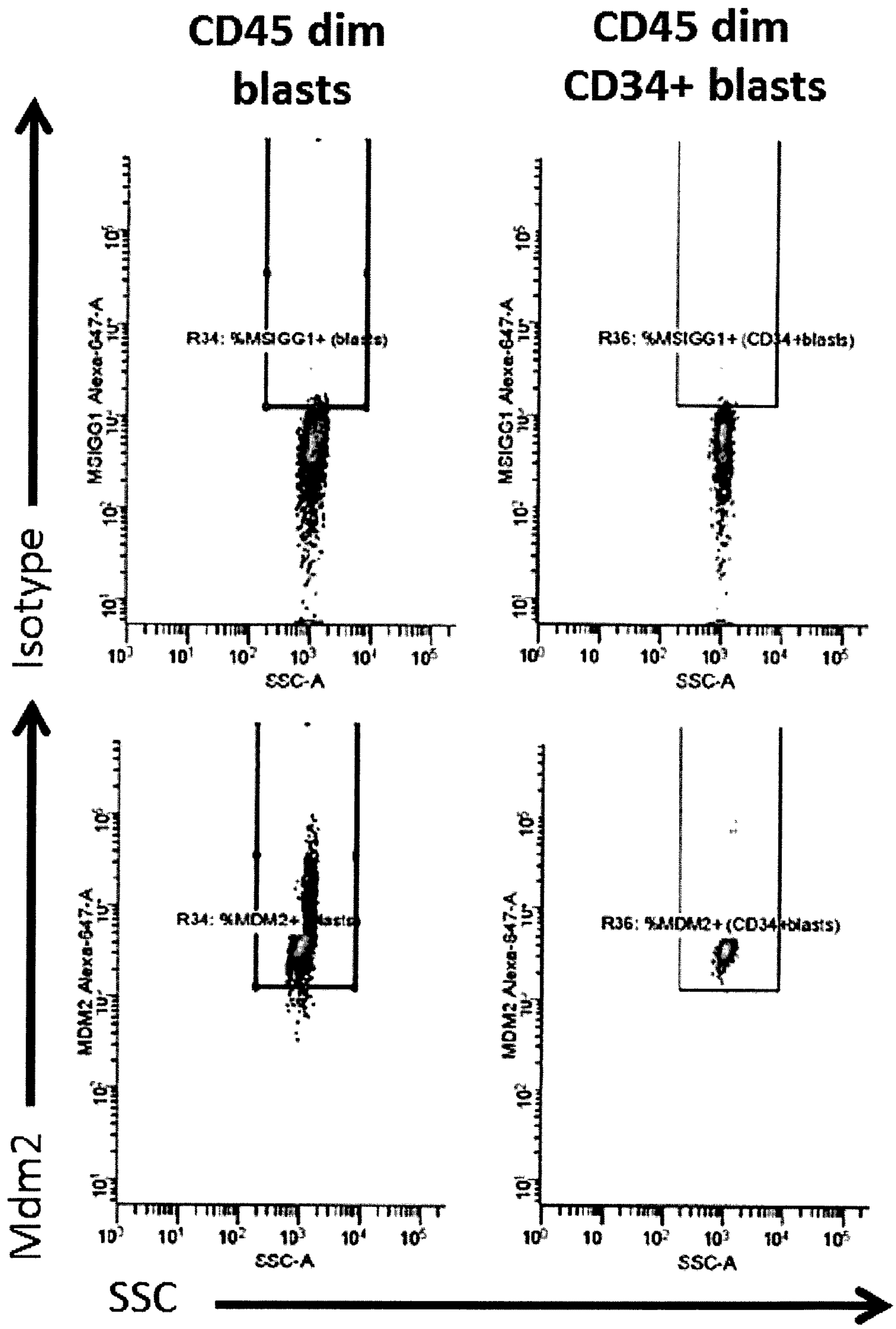
CD45 dim
blasts
CD45 dim
CD34+ blasts
Isotype
Mdm2
SSC
R34: %MSIGG1+ (blasts)
R36: %MSIGG1+ (CD34+blasts)
R34: %MDM2+ blasts
R36: %MDM2+ (CD34+blasts)
MSIGG1 Alexa-647-A
MDM2 Alexa-647-A
SSC-A

Fig. 2

## NP28679 (Part1 and Part2) -

**Mdm2 Expression in CD45dim (Blast) Cells vs Best Overall Response**

(p53 mutants included)

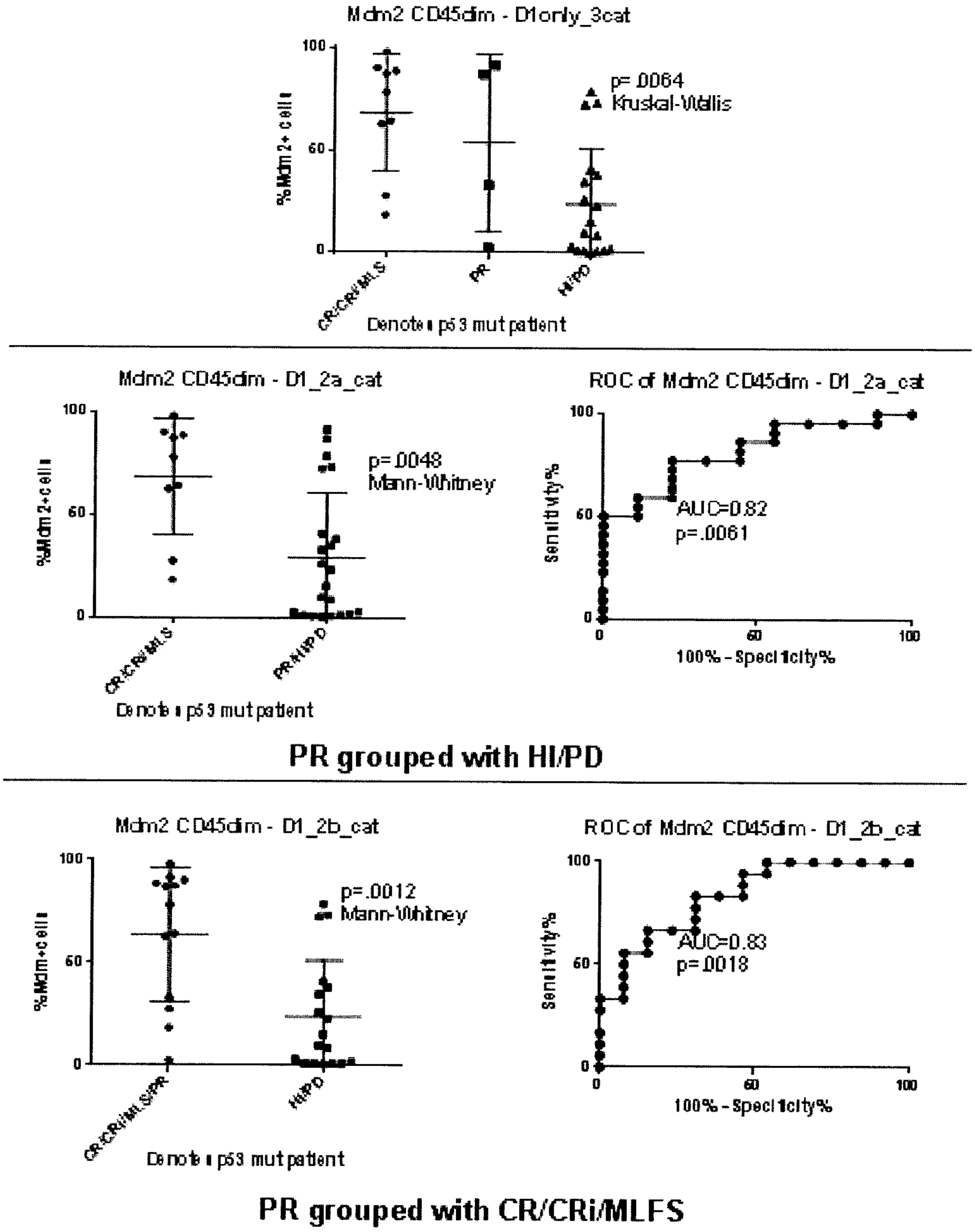


Fig. 3(a)

# NP28679 (Part 1 and Part 2) -

**Mdm2 Expression in CD45dim (Blast) Cells vs Best Overall Response**

(p53 mutants excluded)

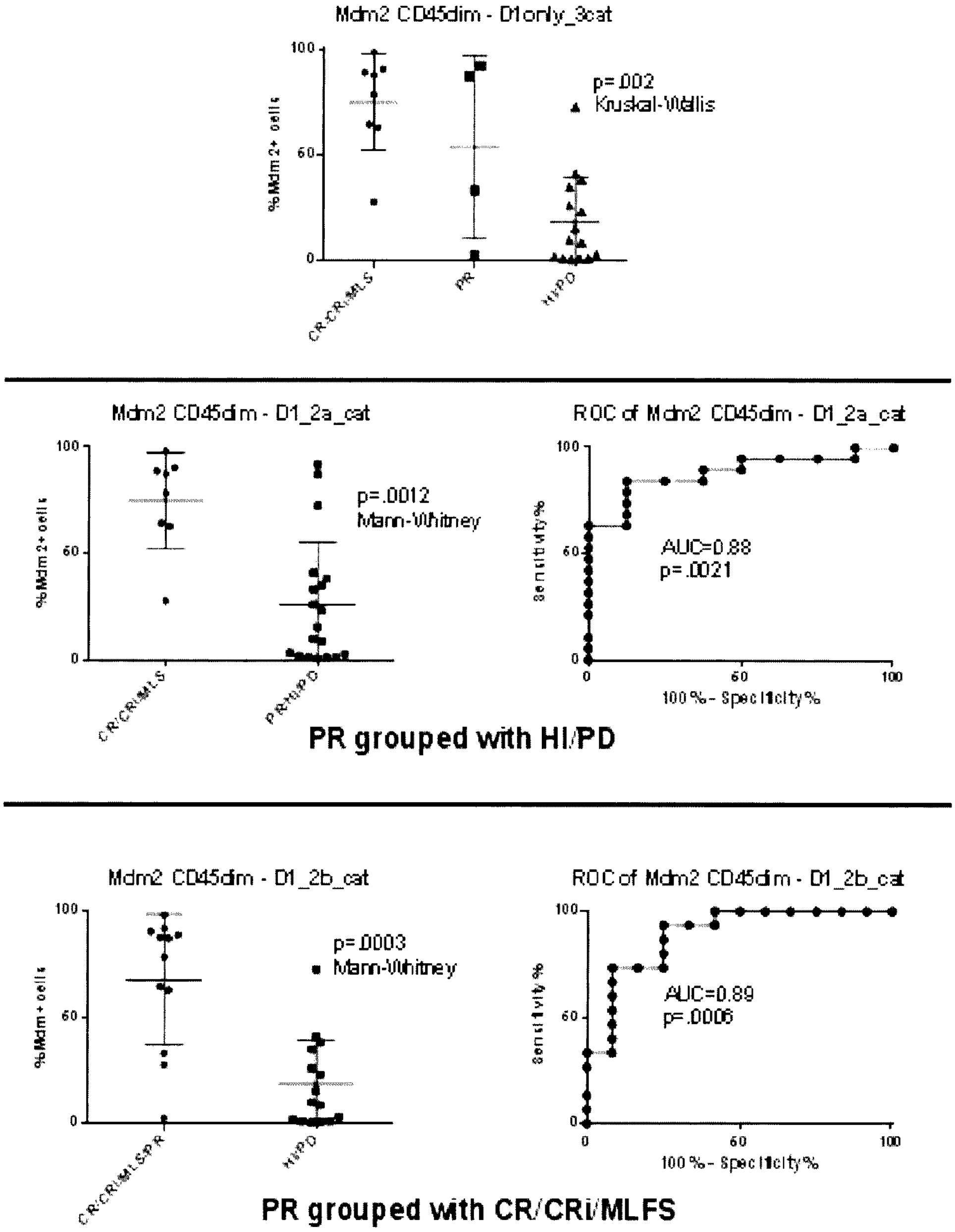


Fig. 3(b)

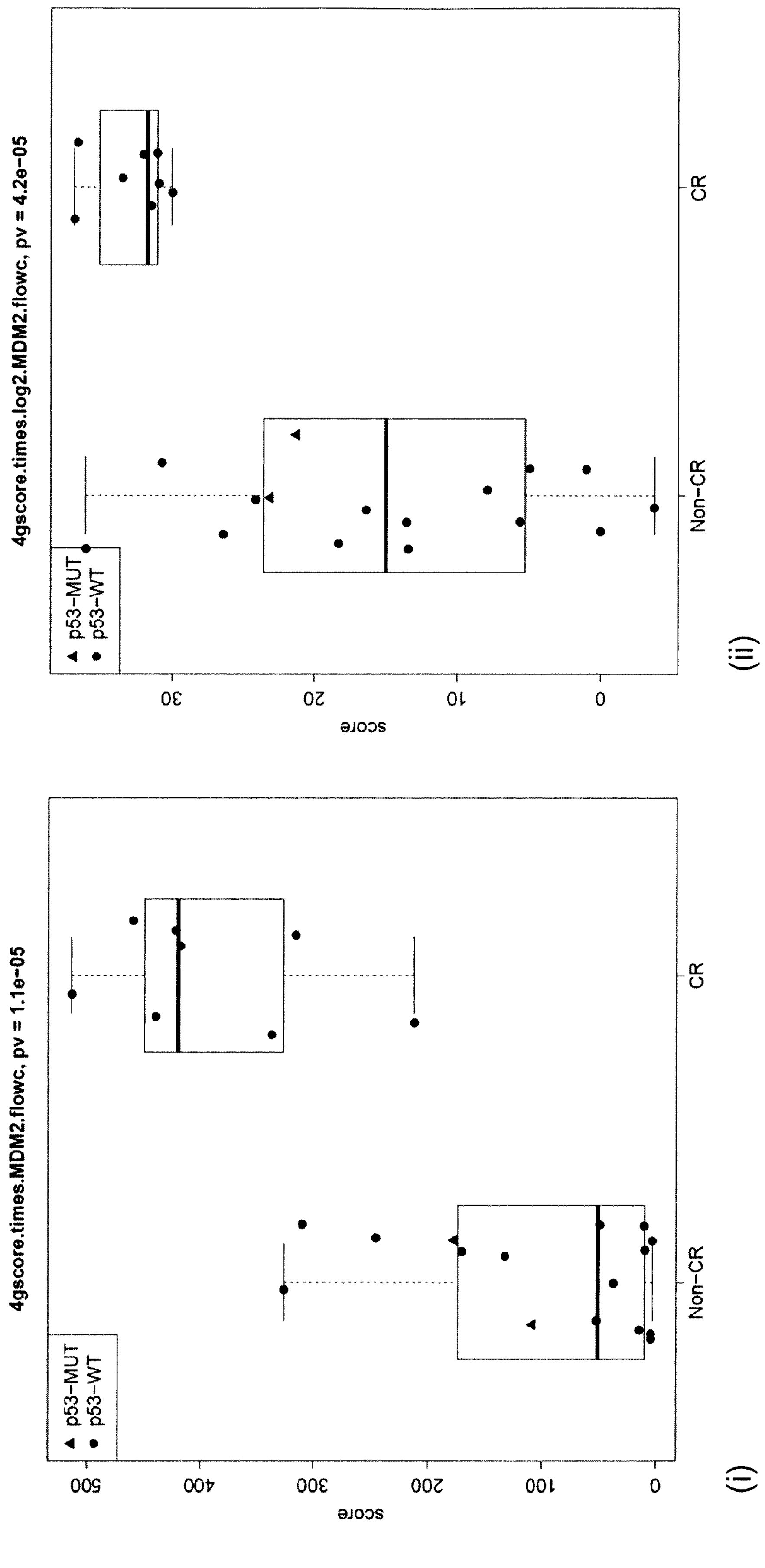
4gscore.times.MDM2.flowc, pv = 1.1e-05
p53-MUT
p53-WT
score
0
100
200
300
400
500
Non-CR
CR
(i)
4gscore.times.log2.MDM2.flowc, pv = 4.2e-05
p53-MUT
p53-WT
score
0
10
20
30
Non-CR
CR
(ii)

Fig. 4a

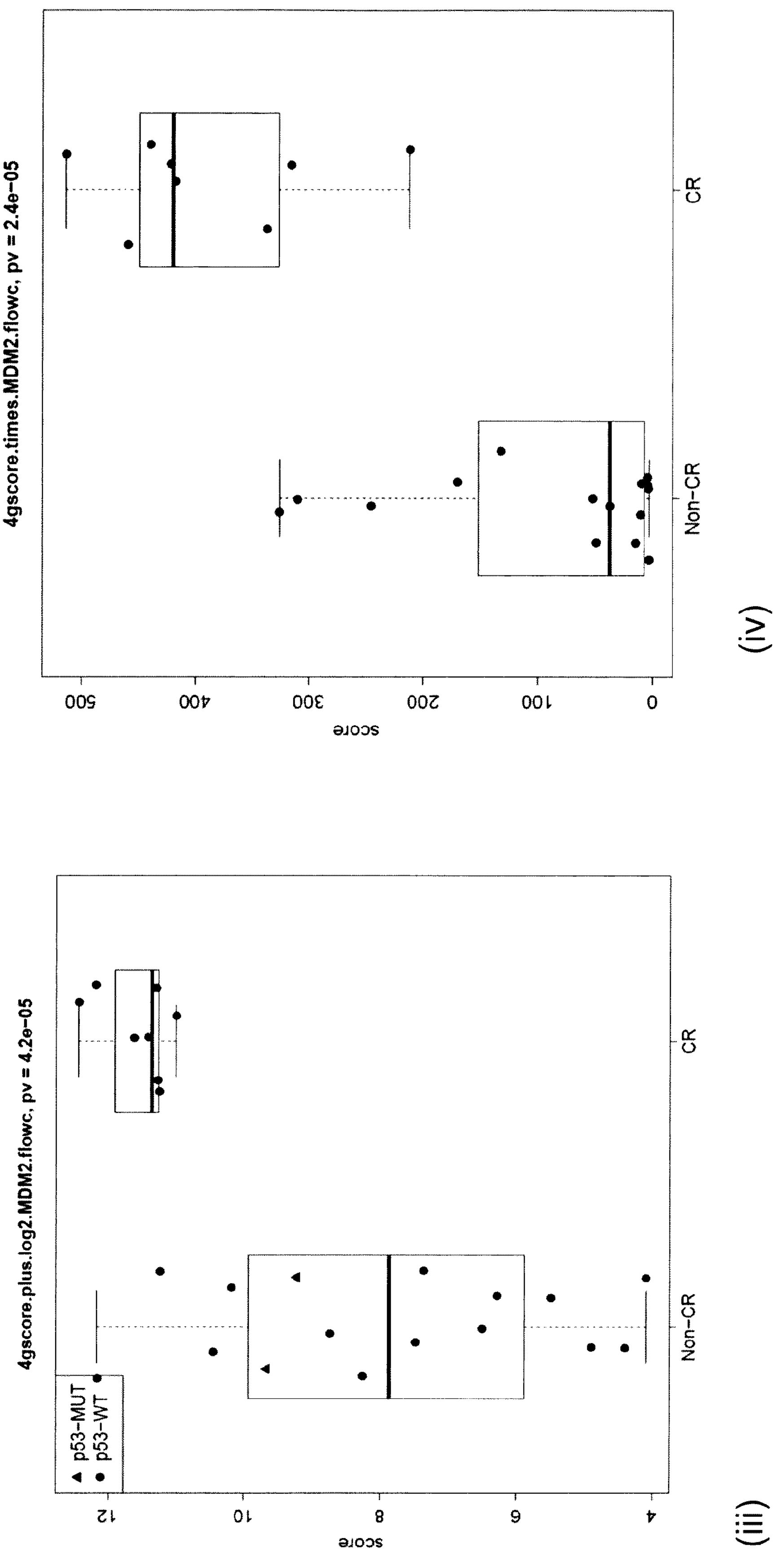
4gscore.plus.log2.MDM2.flowc, pv = 4.2e−05
p53−MUT
p53−WT
score
Non−CR
CR
(iii)
4gscore.times.MDM2.flowc, pv = 2.4e−05
score
Non−CR
CR
(iv)

Fig. 4a

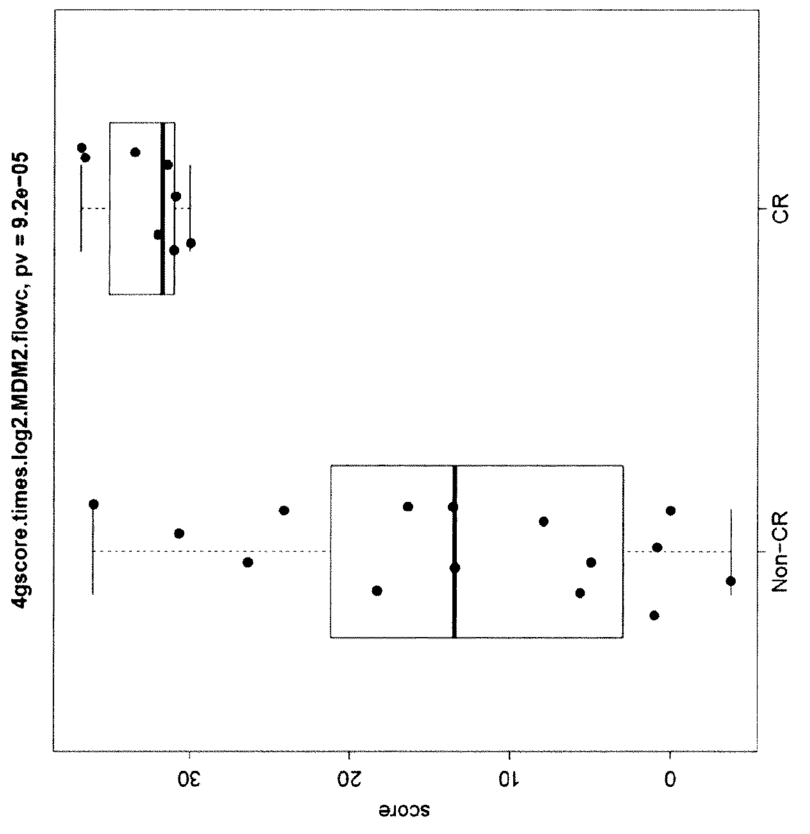
4gscore.times.log2.MDM2.flowc, pv = 9.2e−05
score
0
10
20
30
Non−CR
CR

(v)

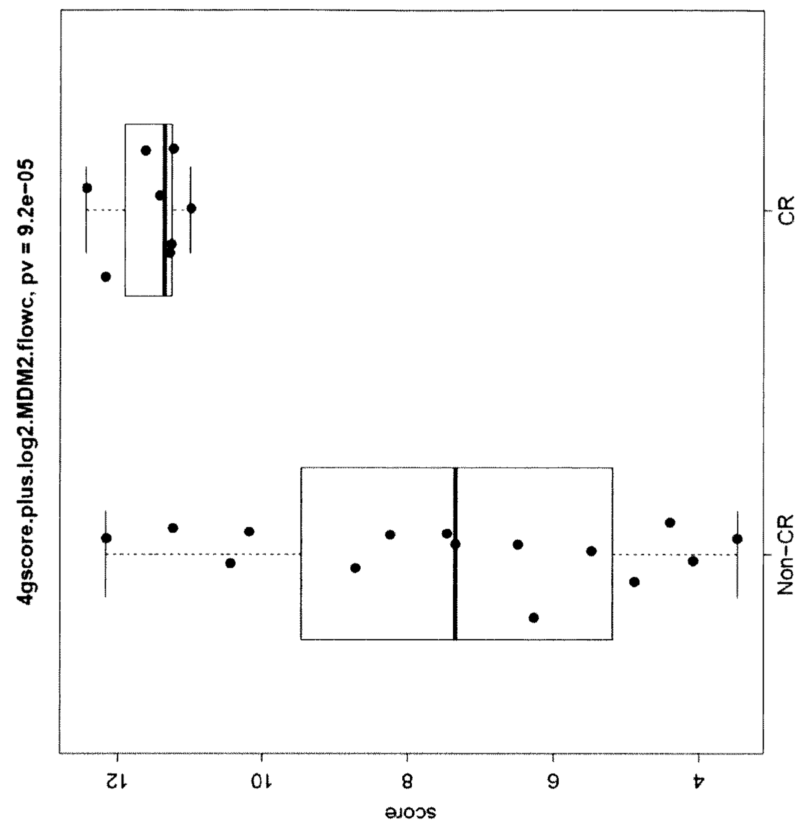
4gscore.plus.log2.MDM2.flowc, pv = 9.2e−05
score
4
6
8
10
12
Non−CR
CR

(vi)

Fig. 4a

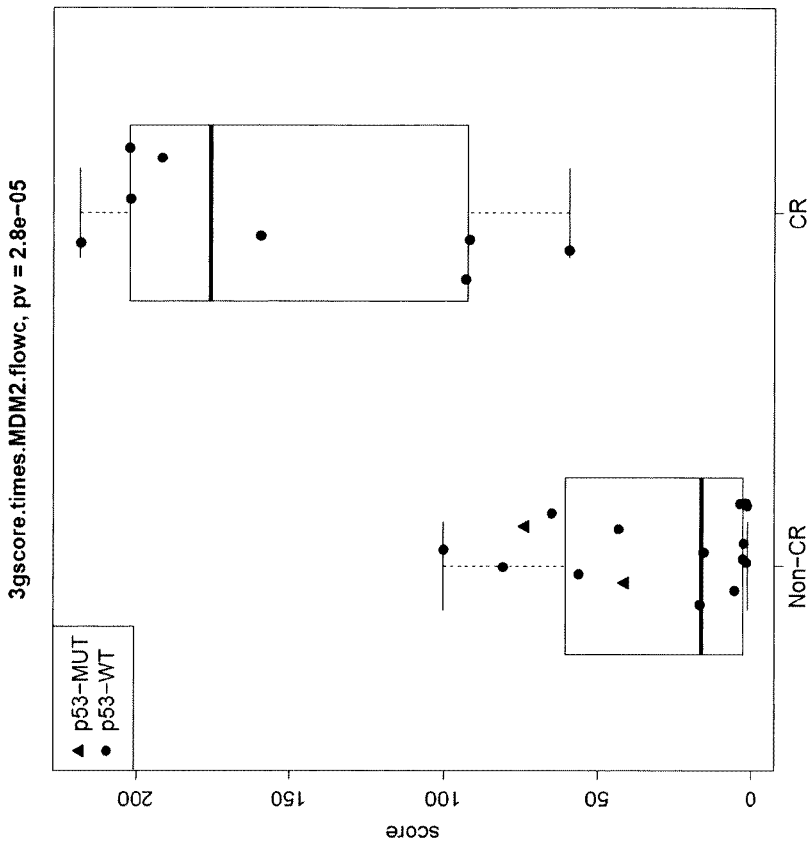
3gscore.times.MDM2.flowc, pv = 2.8e−05
p53−MUT
p53−WT
score
0
50
100
150
200
Non−CR
CR

(ii)

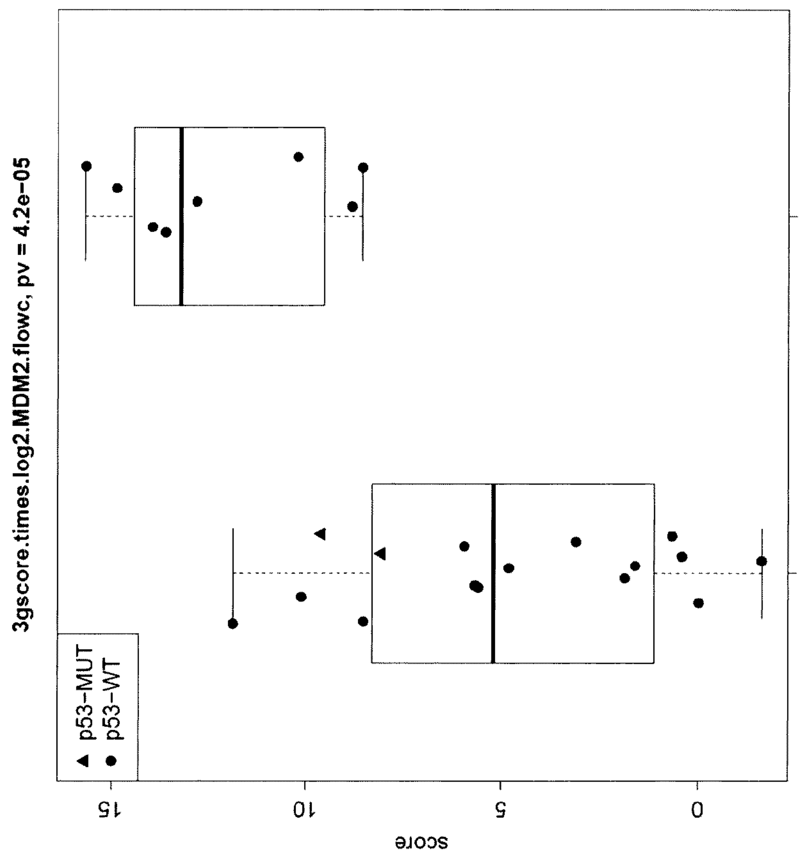
3gscore.times.log2.MDM2.flowc, pv = 4.2e−05
p53−MUT
p53−WT
score
0
5
10
15
Non−CR
CR

(i)

Fig. 4b

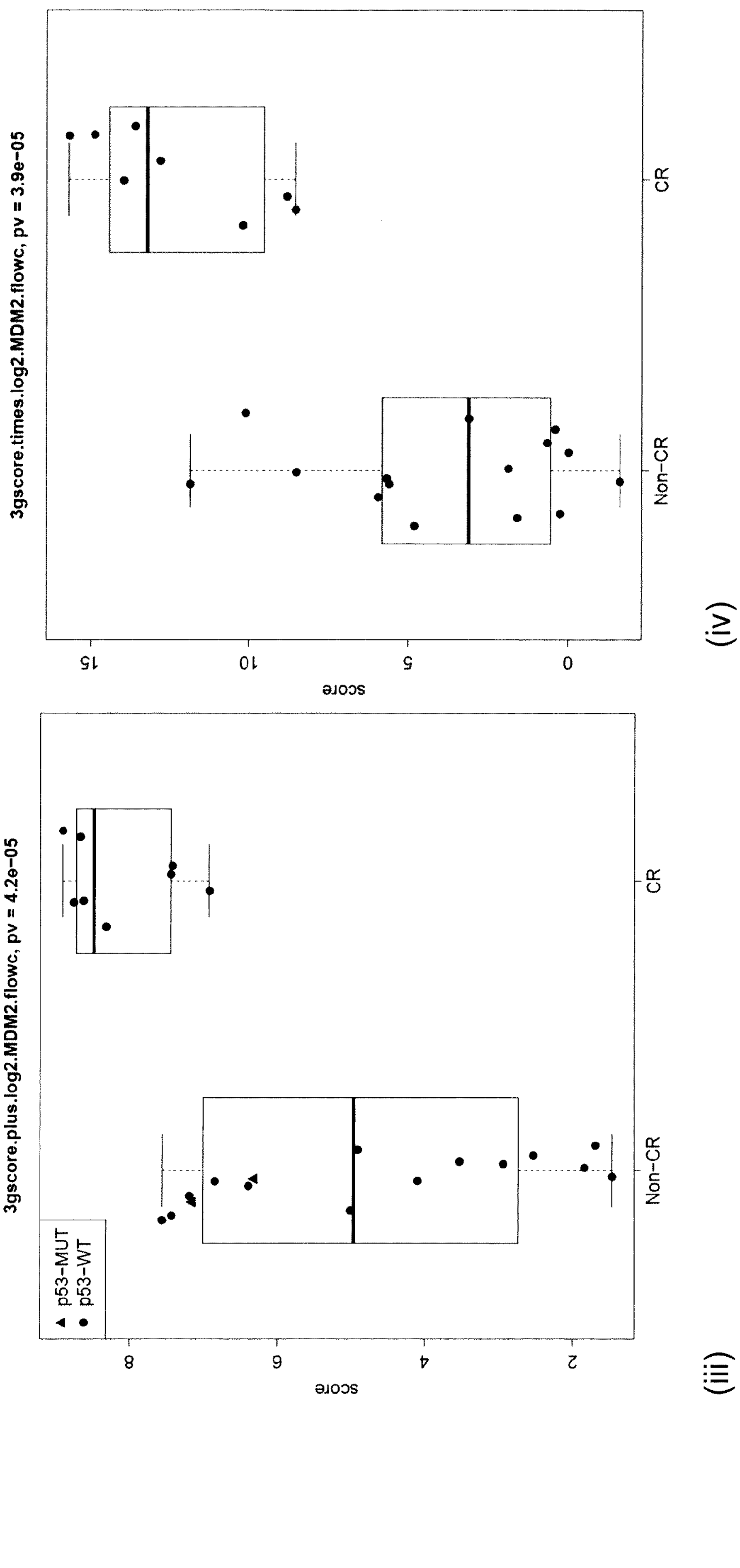
3gscore.plus.log2.MDM2.flowc, pv = 4.2e−05
p53−MUT
p53−WT
score
8
6
4
2
Non−CR
CR
(iii)
3gscore.times.log2.MDM2.flowc, pv = 3.9e−05
score
15
10
5
0
Non−CR
CR
(iv)

Fig. 4b

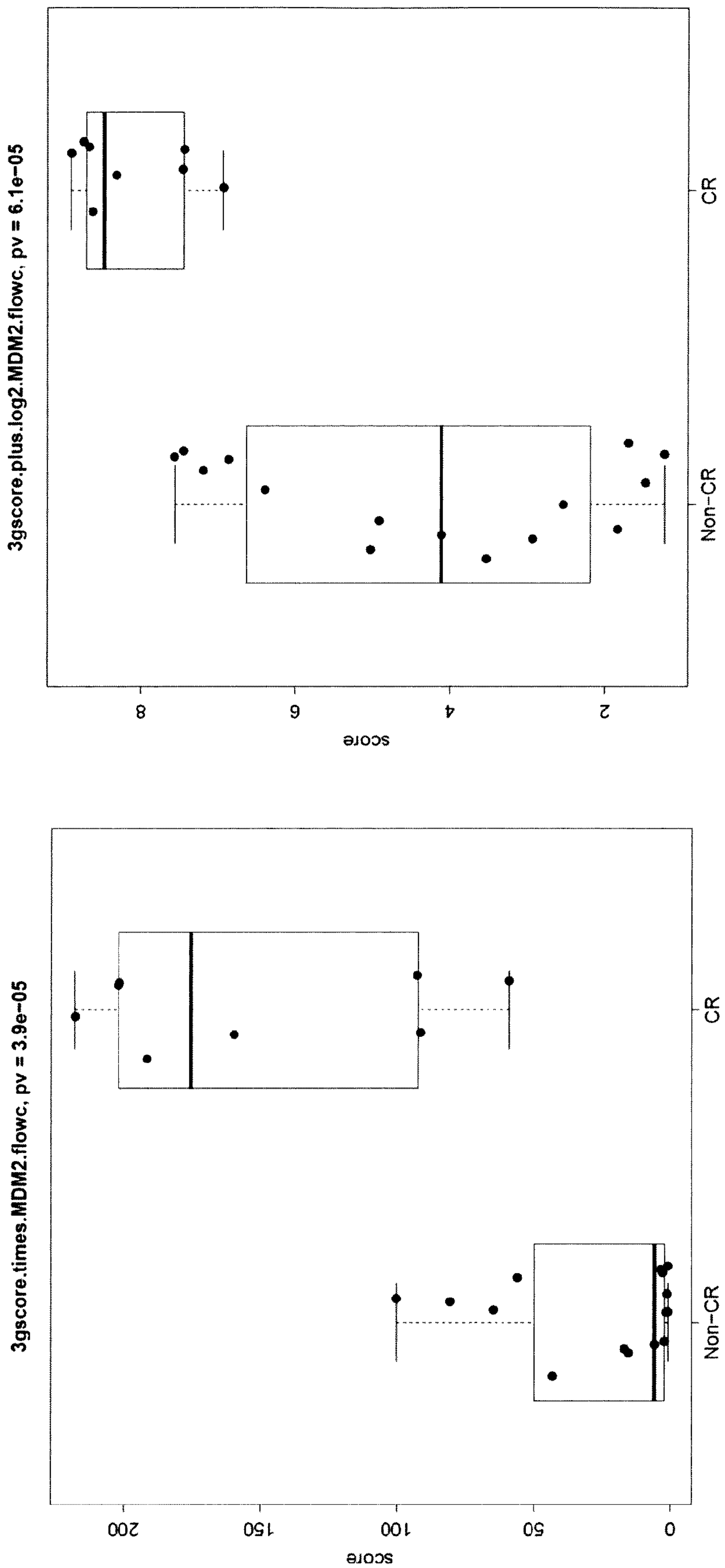
3gscore.times.MDM2.flowc, pv = 3.9e−05
score
0
50
100
150
200
Non−CR
CR
3gscore.plus.log2.MDM2.flowc, pv = 6.1e−05
score
2
4
6
8
Non−CR
CR

(v)

(vi)

Fig. 4b

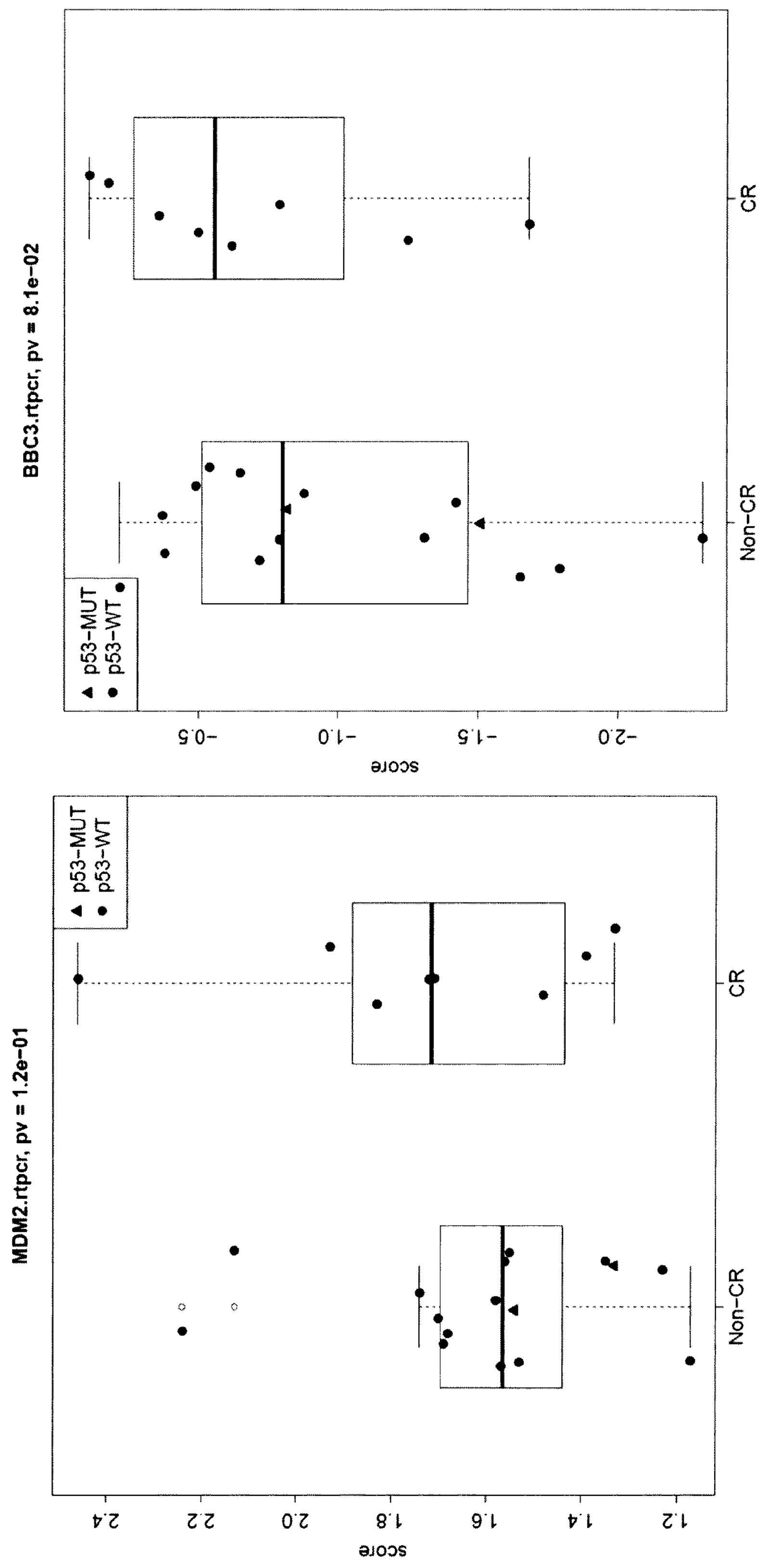
MDM2.rtpcr, pv = 1.2e−01
score
2.4
2.2
2.0
1.8
1.6
1.4
1.2
Non−CR
CR
p53−MUT
p53−WT
BBC3.rtpcr, pv = 8.1e−02
score
−0.5
−1.0
−1.5
−2.0
Non−CR
CR
p53−MUT
p53−WT

(i)

(ii)

Fig. 5

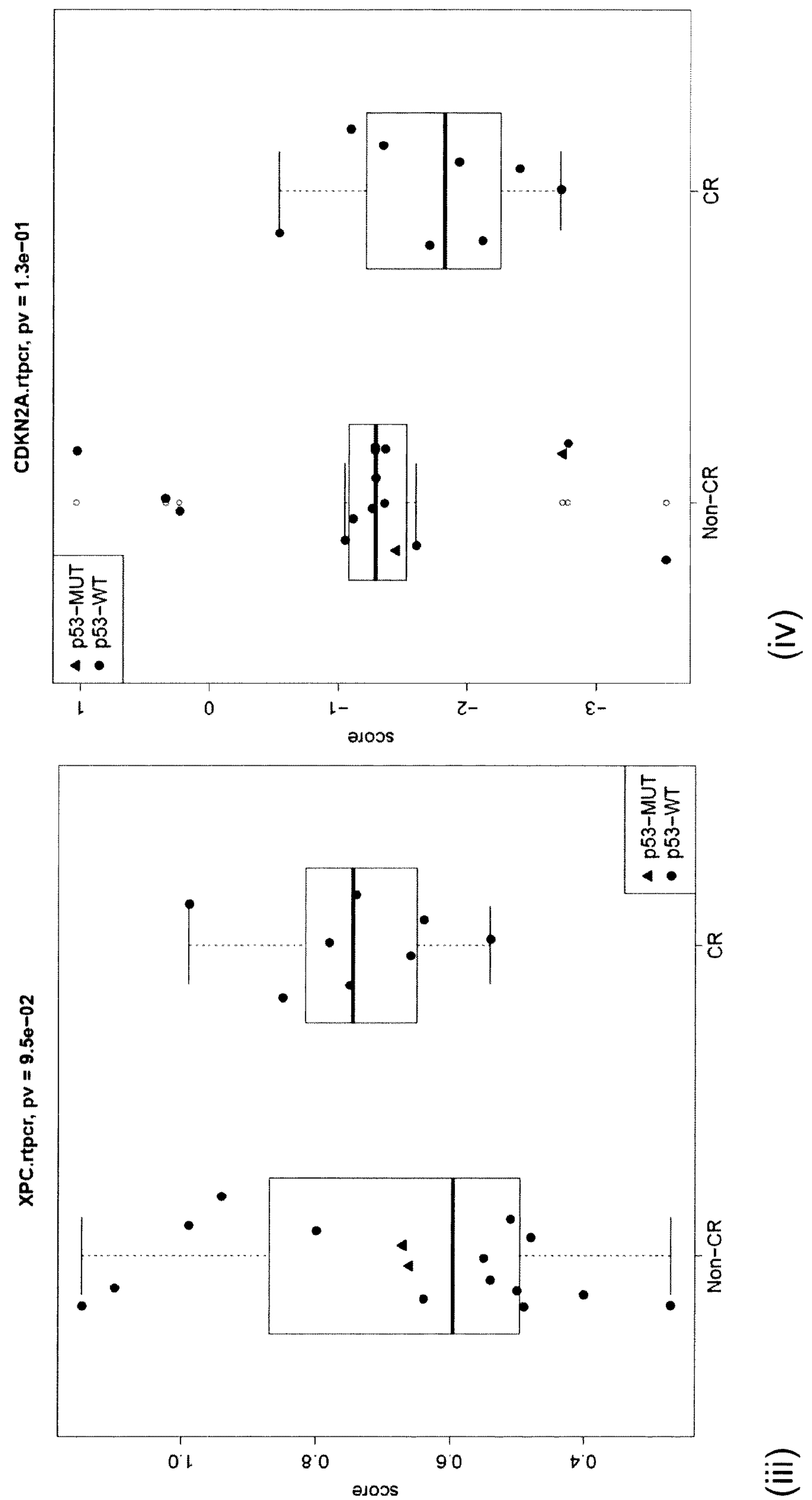
CDKN2A.rtpcr, pv = 1.3e−01
p53−MUT
p53−WT
score
1
0
−1
−2
−3
Non−CR
CR
(iv)
XPC.rtpcr, pv = 9.5e−02
p53−MUT
p53−WT
score
1.0
0.8
0.6
0.4
Non−CR
CR
(iii)

Fig. 5

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2011127058 A
- US 8354444 B2
- WO 2007063013 A
- WO 2010031713 A
- WO 2011098398 A
- WO 2013135648 A
- US 8354444 B
- WO 2014114575 A
- WO 2014206866 A
- WO 2013139687 A
- US 14217929 B
- US 20140200255 A1
- WO 2015000945 A


### Non-patent literature cited in the description


- **ANSEL et al.** Pharmaceutical Dosage Forms and Drug Delivery Systems. 1995, 196, , 1456-1457
- **OKEN, M.M. et al.** *Am. J. Clin. Oncol.,* 1982, 649-655
- **NICHOLAS D. REESE ; GARY J. SCHILLER.** *Curr Hematol Malig Rep,* 2013, vol. 8, 141-148
- **G. MINOTTI et al.** Anthracyclines: Molecular Advances and Pharmacologic Developments in Antitumor Activity and Cardiotoxicity. *Pharmacological Reviews,* 2004, vol. 56 (2), 185-229